(19) Europäisches Patentamt
European Patent Office
Office européen des brevets

(11) EP 4 506 369 A1

(12) **EUROPEAN PATENT APPLICATION**
published in accordance with Art. 153(4) EPC

(43) Date of publication:
**12.02.2025 Bulletin 2025/07**

(21) Application number: **23780883.7**

(22) Date of filing: **30.03.2023**

(51) International Patent Classification (IPC):
***C08B 31/12*** *(2006.01)* ***A61F 13/15*** *(2006.01)*
***A61F 13/53*** *(2006.01)* ***C08B 31/04*** *(2006.01)*

(52) Cooperative Patent Classification (CPC):
**A61F 13/15; A61F 13/53; C08B 31/04; C08B 31/12**

(86) International application number:
**PCT/JP2023/013188**

(87) International publication number:
**WO 2023/190875 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.03.2022 JP 2022058598**

(71) Applicants:
- **Nagase & Co., Ltd.**
  **Osaka-shi, Osaka 550-8668 (JP)**
- **NAGASE CHEMTEX CORPORATION**
  **Osaka-shi, Osaka 550-8668 (JP)**
- **Nagase Viita Co., Ltd.**
  **Okayama-shi, Okayama 702-8006 (JP)**

(72) Inventors:
- **NOZAKI, Takahiro**
  **Tokyo 100-8142 (JP)**
- **TANAKA, Atsushi**
  **Tokyo 100-8142 (JP)**
- **NISHIMOTO, Tomoyuki**
  **Kobe-shi, Hyogo 651-2241 (JP)**
- **HOSOMI, Tetsuya**
  **Tatsuno-shi, Hyogo 679-4124 (JP)**
- **MIYATA, Manabu**
  **Okayama-shi, Okayama 702-8006 (JP)**

(74) Representative: **Mewburn Ellis LLP**
**Aurora Building**
**Counterslip**
**Bristol BS1 6BX (GB)**

(54) **METHOD FOR PRODUCING WATER ABSORBENT RESIN**

(57) The present invention aims to provide a method for producing a water-absorbing resin having excellent water absorption performance with high productivity. The present invention relates to a method for producing a water-absorbing resin that forms a physical gel upon absorption of water, the method including: (a1) reducing the molecular weight of a starch to obtain a partially degraded starch; (a2) introducing an acidic group into the partially degraded starch obtained in (a1) to obtain a water-soluble polymer; and (a3) drying the water-soluble polymer at 70°C to 180°C in the presence of water so that it has a solid content of 90% or more.

EP 4 506 369 A1

## Description

TECHNICAL FIELD

The present invention relates to a method for producing a water-absorbing resin.

BACKGROUND ART

Water-absorbing resins are widely used in various fields such as hygiene products, foods, agriculture and forestry, and civil engineering. As such water-absorbing resins, partially neutralized salts of polyacrylic acids or polymethacrylic acids are widely used. Also known are water-absorbing resins made from polysaccharides such as starch.
Patent Literature 1 discloses a method of producing a water-absorbing resin by heat drying a carboxyalkylated starch to crosslink one starch with another. This document describes that it is preferred to reduce the decrease in the molecular weight of the starch during the carboxyalkylation reaction.
Patent Literature 2 discloses a method of producing a water-absorbing resin by subjecting carboxyalkylated polysaccharide particles to surface treatment with a non-crosslinking acid such as hydrochloric acid followed by heat drying or reaction with a crosslinking agent to crosslink one polysaccharide with another.
Patent Literature 3 discloses a method of producing a water-absorbing material by reacting a starch with a polybasic acid anhydride in an extruder. This document describes that it is preferred to reduce the decrease in the molecular weight of the starch during the reaction with the polybasic acid anhydride.

CITATION LIST

- Patent Literature

Patent Literature 1: U.S. Patent No. 5079354
Patent Literature 2: JP 2010-504414 T
Patent Literature 3: JP 2007-222704 A

SUMMARY OF INVENTION

- Technical Problem

Conventional water-absorbing resins made from polysaccharides do not have sufficient water absorption performance. Moreover, since such water-absorbing resins have been produced from high-molecular weight polysaccharides, they have a handleability problem during the production due to the highly viscous raw materials. An object of the present invention is to provide a method for producing a water-absorbing resin having excellent water absorption performance with high productivity.

- Solution to Problem

The present inventors have focused on the molecular weight of a starch to be used as a raw material of a water-absorbing resin as well as the cross-linking conditions thereof. Then, they have completed the present invention.
Specifically, the present invention relates to a method for producing a water-absorbing resin that forms a physical gel upon absorption of water, the method including:

(a1) reducing a molecular weight of a starch to obtain a partially degraded starch;
(a2) introducing an acidic group into the partially degraded starch obtained in (a1) to obtain a water-soluble polymer; and
(a3) drying the water-soluble polymer at 70°C to 180°C in the presence of water so that it has a solid content of 90% or more.

Preferably, the method includes, after (a2),
(aa) desalting the water-soluble polymer.
Preferably, the method includes, in (a2) or after (a2) but before (a3),
(ab) partially neutralizing the acidic group in the water-soluble polymer.

Preferably, the method does not include, after (a2) but before (a3), partially neutralizing the acidic group in the water-soluble polymer.

Preferably, the method includes, in (a3), drying the water-soluble polymer so that it has a solid content of at least 90% but not more than 99%.

Preferably, the acidic group is a carboxyalkyl group, a carboxyalkenyl group, or a sulfoalkyl group.

Preferably, the method includes, in (a1), obtaining the partially degraded starch that has at least one of a weight average molecular weight (Mw) of 7,500,000 or less or a dispersity (weight average molecular weight (Mw)/number average molecular weight (Mn)) of 5 or more.

Preferably, the method includes, in (a1), reducing the molecular weight of the starch by enzymatic treatment.

Preferably, the method includes, in (a2), introducing the acidic group into a mixture of the partially degraded starch obtained in (a1) and a starch whose molecular weight has not been reduced.

Preferably, the water-soluble polymer obtained in (a2) has a weight average molecular weight (Mw) of 500,000 to 40,000,000 as determined by aqueous size exclusion chromatography using pullulan standards.

Preferably, the water-absorbing resin to be produced has at least one of the following characteristics:

(a) a water absorption rate under no pressure for ion-exchanged water is 100 to 500 g/g;
(b) a water retention rate for ion-exchanged water is 80 to 300 g/g;
(c) a water absorption rate under no pressure for physiological saline is 10 to 70 g/g; and
(d) a water retention rate for physiological saline is 5 to 65 g/g.

- Advantageous Effects of Invention

The method for producing a water-absorbing resin of the present invention can produce a water-absorbing resin having excellent water absorption performance with high productivity.

## BRIEF DESCRIPTION OF DRAWING

FIG. 1 shows photographs of gelled water-absorbing resins.

## DESCRIPTION OF EMBODIMENTS

### <<Method 1 for producing water-absorbing resin>>

The method for producing a water-absorbing resin of the present invention produces a water-absorbing resin that forms a physical gel upon absorption of water and includes: (a1) reducing the molecular weight of a starch to obtain a partially degraded starch; (a2) introducing an acidic group into the partially degraded starch obtained in (a1) to obtain a water-soluble polymer; and (a3) drying the water-soluble polymer at 70°C to 180°C in the presence of water so that it has a solid content of 90% or more.

### <Step (a1) of reducing molecular weight of starch to obtain partially degraded starch>

In this step, the molecular weight of a starch is reduced to decrease the viscosity. The type of the starch as a raw material is not limited, and examples include waxy corn starch, tapioca starch, potato starch, corn starch (including waxy corn starch and high amylose starch), wheat starch, rice starch, and sweet potato starch.

The starch may be a bleached starch or a derivative obtained by partially replacing the hydrogen atoms of the hydroxyl groups of the glucose units with a functional group (also referred to as a modified starch or a chemically modified starch). The position of the hydroxyl group whose hydrogen atom is replaced may be any of the 1-, 2-, 3-, 4-, and 6-positions, preferably any of the 2-, 3-, and 6-positions in the glucose unit. Examples of the functional group include hydrocarbon groups such as a methyl group and an ethyl group; hydroxyl group-containing substituents such as a hydroxypropyl group and a hydroxyethyl group; carboxyl group-containing substituents such as a carboxymethyl group; and acyl groups such as a formyl group, an acetyl group, and a propionyl group.

Specific examples of the derivative of the starch include what is called modified starch used in foods or for industrial purposes, such as acetylated starch, acetylated distarch adipate, acetylated distarch phosphate, acetylated oxidized starch, starch sodium octenyl succinate, starch acetate, oxidized starch, hydroxypropyl starch, hydroxypropyl distarch phosphate, phosphated distarch phosphate, monostarch phosphate, distarch phosphate, cationized starch, and urea phosphate esterified starch. Methyl-ethylated starch, hydroxypropyl-methylated starch, and other starches substituted with two or more functional groups are also usable. Among these, starch acetate, oxidized starch, acetylated starch, acetylated oxidized starch, hydroxypropyl starch, hydroxypropyl distarch phosphate, and starch sodium octenyl

succinate are preferred to easily control the properties of the water-absorbing resin.

The method for reducing the molecular weight of the starch is not limited, and examples include enzymatic treatment, acid treatment, physical crushing, etc. These methods may also be used in combination. In these methods, it is preferred to partially hydrolyze the glucoside bonds of the α-glucose molecules constituting a starch, although the position and mode of degradation are not limited. The reactor used may be a reaction vessel or an extruder, for example.

When the molecular weight of a starch is reduced by enzymatic treatment, the enzyme to be used is preferably, but not limited to, an endo-enzyme in order to efficiently reduce the molecular weight. Specific examples of the enzyme include α-amylase, cyclomaltodextrin glucanotransferase, 4-α-glucanotransferase, 6-α-glucanotransferase, 4,6-α-glucanotransferase, amylomaltase, neopullulanase, amylopullulanase, etc. These enzymes may be used in combination. The pH during the enzymatic treatment is not limited, but it is preferably pH 5.0 to 7.0. The pH can be adjusted by adding hydrochloric acid, acetic acid, sodium hydroxide, potassium hydroxide, or the like. The enzymatic treatment may be performed while heating and kneading the starch at 70°C to 110°C to gelatinize it. The enzymatic treatment may also be performed after or at the same time as the gelatinization of the starch. Examples of the method for performing the enzymatic treatment after the gelatinization of the starch include a method including first suspending and heating a starch in water to gelatinize it, followed by adding an enzyme thereto to perform an enzymatic reaction. Moreover, examples of the method for performing the enzymatic treatment at the same time as the gelatinization of the starch include a method including suspending a starch in water and adding an enzyme thereto to give a liquid mixture, followed by heating the liquid mixture within a temperature range that does not completely inactivate the enzyme.

When the molecular weight of a starch is reduced by acid treatment, specific examples of the acid to be used include, but are not limited to, hydrochloric acid, sulfuric acid, oxalic acid, acetic acid, formic acid, trifluoroacetic acid, etc. The temperature during the acid treatment is preferably 150°C to 160°C.

When a starch is partially degraded by physical crushing, examples of specific means include radiation, shearing, trituration, high-pressure treatment, ultrasonication, heat degradation, photodegradation, and combinations thereof.

The partially degraded starch obtained in the step (a1) preferably has a weight average molecular weight of 7,500,000 or less, more preferably 6,000,000 or less. If the weight average molecular weight is more than 7,500,000, the partially degraded starch has a higher viscosity, which tends to reduce the reaction during the introduction of an acidic group in the step (a2) or the handleability during the purification. The lower limit of the weight average molecular weight of the partially degraded starch is not limited, but it is preferably 50,000 or more, more preferably 200,000 or more. If the weight average molecular weight is less than 50,000, the water retention properties of the water-absorbing resin tend to decrease. Here, the method for measuring the weight average molecular weight is not limited. For example, the weight average molecular weight can be determined by aqueous size exclusion chromatography relative to a calibration curve of molecular weight vs. elution time prepared using pullulans with known molecular weights.

The number average molecular weight of the partially degraded starch obtained in the step (a1) is not limited, but in consideration of the viscosity, it is preferably 1,000,000 or less. Moreover, the lower limit of the number average molecular weight of the partially degraded starch is preferably 10,000 or more, more preferably 50,000 or more. Here, the method for measuring the number average molecular weight is not limited. For example, the number average molecular weight can be determined by aqueous size exclusion chromatography relative to a calibration curve of molecular weight vs. elution time prepared using pullulans with known molecular weights.

The weight average molecular weight or number average molecular weight of the partially degraded product obtained in the step (a1) may also be controlled by mixing two or more partially degraded starches. In this case, the weight average molecular weight or number average molecular weight of the mixture preferably satisfies the numerical range described above.

The dispersity (weight average molecular weight/number average molecular weight) of the partially degraded starch obtained in the step (a1) is preferably 5 or more, more preferably 7 or more. The upper limit of the dispersity is not limited as long as the numerical range of the weight average molecular weight described above is satisfied, but the upper limit is usually 70 or less.

The starch whose molecular weight has not been reduced may remain in the step (a1). The amount of the remaining starch whose molecular weight has not been reduced, if present, is preferably less than 20% by weight relative to the total amount of the starch charged in the step (a1).

<Step (a2) of obtaining water-soluble polymer>

In this step, an acidic group is introduced into the partially degraded starch obtained in the step (a1) to obtain a water-soluble polymer. The water-soluble polymer includes the partially degraded starch with an acidic group introduced therein. The introduced acidic group may be in any of the free acid, salt-forming, or ionized states. These states may coexist.

Also, in this step, an acidic group may be introduced into a mixture of the partially degraded starch obtained in the step (a1) and a starch whose molecular weight has not been reduced. The starch whose molecular weight has not been

reduced may be the starch which has been subjected to the molecular weight-reducing reaction in the step (a1) but has not been degraded to a weight average molecular weight (Mw) of 7,500,000 or less, or may be an untreated starch added after the step (a1). The presence of the starch whose molecular weight has not been reduced can increase the water absorption amount of the produced water-absorbing resin. This is probably because the presence of a water-soluble polymer derived from the starch whose molecular weight has not been reduced can increase the distance between the cross-linking points of the resulting physical gel, thereby facilitating the spread of the cross-linked network. In this step, two or more starches and/or partially degraded starches with different weight average molecular weights may be used together depending on the application or allowable cost.

Examples of the acidic group include carboxyl group-containing acidic groups such as carboxyalkyl groups and carboxyalkenyl groups; sulfo group-containing acidic groups such as sulfoalkyl groups and sulfoalkenyl groups; and phospho group-containing acidic groups such as phosphoalkyl groups and phosphoalkenyl groups.

A carboxyalkyl group refers to an alkyl group substituted with a carboxyl group. The number of carbon atoms of the alkyl group to be substituted with a carboxyl group is preferably 1 to 8, more preferably 1 to 5. The alkyl group may be either linear or branched. Specific examples of the alkyl group include a methyl group, an ethyl group, an n-propyl group, an i-propyl group, an n-butyl group, an i-butyl group, an s-butyl group, a t-butyl group, an n-pentyl group, a 1-methyl-n-butyl group, a 2-methyl-n-butyl group, a 3-methyl-n-butyl group, a 1,1-dimethyl-n-propyl group, a 1,2-dimethyl-n-propyl group, a 2,2-dimethyl-n-propyl group, a 1-ethyl-n-propyl group, etc.

Specific examples of such carboxyalkyl groups include a carboxymethyl group, a carboxyethyl group, a carboxypropyl group, a carboxybutyl group, a carboxypentyl group, etc.

A carboxyalkenyl group refers to an alkenyl group substituted with a carboxyl group. The number of carbon atoms of the alkenyl group to be substituted with a carboxyl group is preferably 2 to 8, more preferably 2 to 4. The alkenyl group may be either linear or branched. Specific examples of the alkenyl group include an ethenyl group, a 1-propenyl group, a 2-propenyl group, a 1-methyl-1-ethenyl group, a 1-butenyl group, a 2-butenyl group, a 3-butenyl group, a 2-methyl-1-propenyl group, a 2-methyl-2-propenyl group, a 1-ethylethenyl group, a 1-methyl-1-propenyl group, a 1-methyl-2-propenyl group, etc.

Specific examples of such carboxyalkenyl groups include a carboxyethenyl group, a carboxypropenyl group, a carboxybutenyl group, etc.

A sulfoalkyl group refers to an alkyl group substituted with a sulfo group. Examples of the alkyl group to be substituted with a sulfo group include the alkyl groups listed for the carboxyalkyl groups. Specific examples of such sulfoalkyl groups include a sulfomethyl group, a sulfoethyl group, a sulfopropyl group, etc.

A sulfoalkenyl group refers to an alkenyl group substituted with a sulfo group. Examples of the alkenyl group to be substituted with a sulfo group include the alkenyl groups listed for the carboxyalkenyl groups. Specific examples of such sulfoalkenyl groups include a sulfoethenyl group, a sulfopropenyl group, etc.

A phosphoalkyl group refers to an alkyl group substituted with a phospho group. Examples of the alkyl group to be substituted with a phospho group include the alkyl groups listed for the carboxyalkyl groups. Specific examples of such phosphoalkyl groups include a phosphomethyl group, a phosphoethyl group, a phosphopropyl group, etc.

A phosphoalkenyl group refers to an alkenyl group substituted with a phospho group. Examples of the alkenyl group to be substituted with a phospho group include the alkenyl groups listed for the carboxyalkenyl groups. Specific examples of such phosphoalkenyl groups include a phosphoethenyl group, a phosphopropenyl group, etc.

Among these acidic groups, carboxyl group- or sulfo group-containing acidic groups are preferred, with carboxyalkyl groups, carboxyalkenyl groups, and sulfoalkyl groups being more preferred, with carboxyalkyl groups having 1 to 5 carbon atoms being still more preferred.

In order to introduce an acidic group, the partially degraded starch may be reacted with an acidic group-containing compound or a precursor thereof. The acidic group-containing compound is not limited as long as it can introduce an acidic group as described above. Examples include acidic group-containing haloalkyl compounds, acidic group-containing haloalkenyl compounds, acid anhydrides, and salts thereof. Examples of the halogens constituting the haloalkyl compounds or haloalkenyl compounds include chlorine and bromine.

Specific examples of the acidic group-containing compound include monochloroacetic acid, monobromoacetic acid, 3-bromopropionic acid, 6-bromohexanoic acid, succinic anhydride, maleic anhydride, vinylsulfonic acid, phosphorus oxychloride, ethyl monochloroacetate, and sodium or potassium salts thereof, etc.

Examples of the precursor of the acidic group-containing compound include acrylonitrile. Acrylonitrile may be used, for example, by first reacting acrylonitrile with a starch or a partially degraded product thereof under basic conditions to introduce a cyanoethyl group, deriving an amide group from the cyanoethyl group (Synthesis; 1989(12):949-950), and then subjecting the resulting amide to alkaline hydrolysis.

The reaction of the partially degraded starch with monochloroacetic acid to produce a water-soluble polymer is summarized in formula (I).

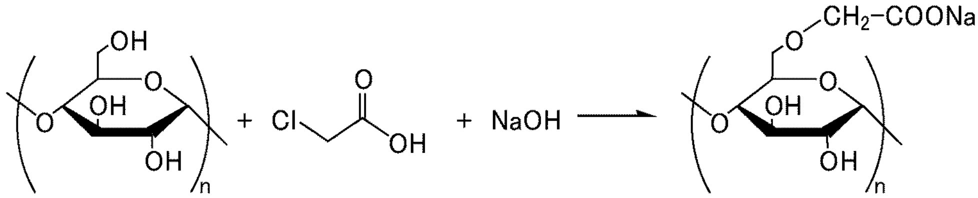

(I)

The reaction of the partially degraded starch with 3-bromopropionic acid to produce a water-soluble polymer is summarized in formula (II).

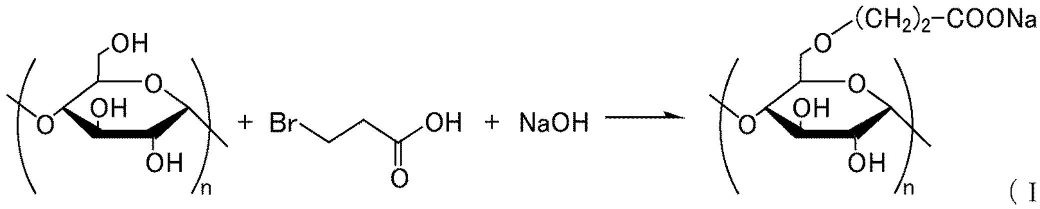

(II)

The reaction of the partially degraded starch with 6-bromohexanoic acid to produce a water-soluble polymer is summarized in formula (III).

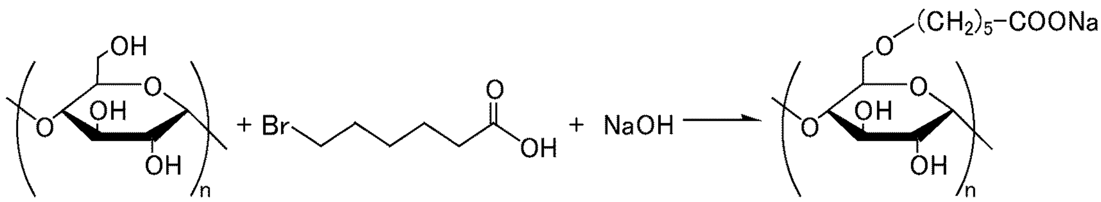

(III)

The reaction of the partially degraded starch with succinic anhydride to produce a water-soluble polymer is summarized in formula (IV).

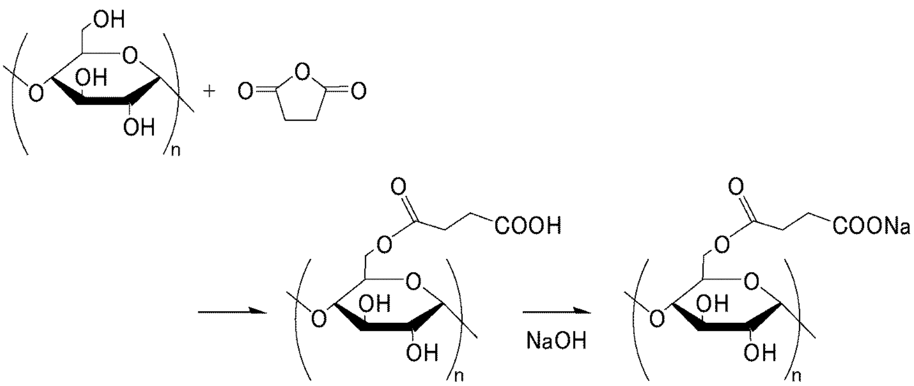


(IV)

The reaction of the partially degraded starch with maleic anhydride to produce a water-soluble polymer is summarized in formula (V).

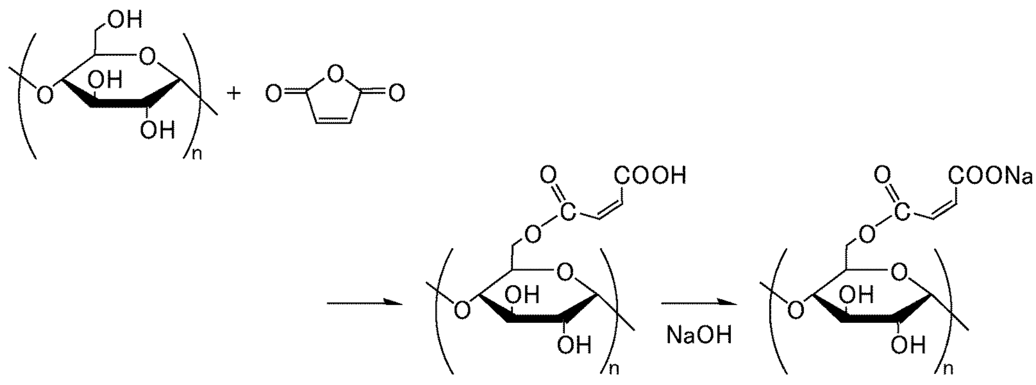


(V)

The reaction of the partially degraded starch with sodium vinylsulfonate to produce a water-soluble polymer is summarized in formula (VI).

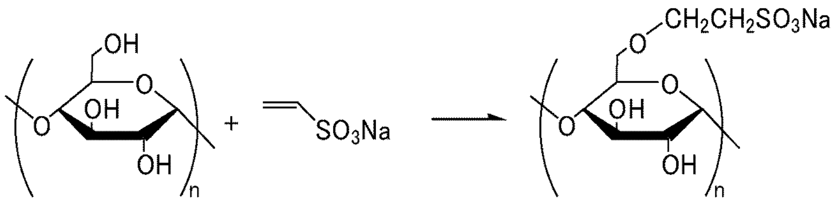

(VI)

Formulas (I) to (VI) show water-soluble polymers in which a sodium salt of an acidic group has been introduced into all hydroxyl groups at the 6-position of the glucose units, but there may remain a hydroxyl group with no acidic group introduced thereinto. There may also be an acidic group that has not been neutralized by a salt. The acidic group can be introduced into a hydroxyl group at any position as long as the hydroxyl group is present in the partially degraded starch, and the hydroxyl group may be at any of the 1-, 2-, 3-, 4-, and 6-positions.

The conditions of the reaction between the partially degraded starch and the acidic group-containing compound are not limited. When the acidic group-containing compound used is a carboxyl group-containing haloalkyl compound, 1 to 1.5 equivalents of an alkaline agent relative to the acidic group-containing compound is preferably used. When the acidic group-containing compound used is a carboxyl group (acidic group)-containing haloalkyl compound, such as monochloroacetic acid or monobromoacetic acid, an alkaline agent is preferably used in an amount required for neutralization of the carboxyl group, i.e., in an amount equivalent to the carboxyl group. Examples of the alkaline agent include sodium hydroxide, potassium hydroxide, lithium hydroxide, ammonia, sodium carbonate, potassium carbonate, etc. These alkaline agents may be used alone or in combinations of two or more. The pH of the reaction between the partially degraded starch and the acidic group-containing compound or precursor thereof is preferably 4 or more, more preferably 8 or more.

The bonding mode between the partially degraded starch and the acidic group is preferably a covalent bond. Specific examples include an ether bond formed between the hydroxyl group on the partially degraded starch and the alkyl group moiety of the acidic group, as well as an ester bond.

The acidic group that has been introduced into the partially degraded starch preferably forms a salt with sodium, potassium, lithium, ammonia, or the like derived from the alkaline agent. Thus, the alkaline agent is preferably used in an amount required for both the reaction between the acidic group-containing compound and the partially degraded starch and the neutralization of the acidic group of the acidic group-containing compound. For example, when the acidic group-containing compound used is monochloroacetic acid, the alkaline agent is preferably used theoretically in an amount of at least two equivalents relative to the monochloroacetic acid. When sodium monochloroacetate is used, the alkaline agent is preferably used in an amount of at least one equivalent relative to the sodium monochloroacetate because the acidic group has already been neutralized.

The amount of the acidic group-containing compound to be used can be arbitrarily set depending on the desired total acid value (degree of etherification) of the water-soluble polymer. Usually, the amount is preferably 0.5 to 5.0 equivalents, more preferably 0.5 to 2.0 equivalents per mole of hydroxyl groups of the partially degraded starch. When a haloalkyl compound such as monochloroacetic acid in the form of an aqueous solution is to be reacted, the haloalkyl compound is required in excess of the theoretical amount because the introduction reaction of the acidic group will compete with the hydrolysis reaction of the haloalkyl compound. The amount of the haloalkyl compound to be used in the

reaction of the aqueous solution is preferably set to be 5 equivalents or less relative to the theoretical value.

When the acidic group-containing compound used is a haloalkyl compound or a salt thereof, the temperature during the reaction with the partially degraded starch is preferably 0°C to 100°C. The reaction is preferably performed at 25°C to 90°C to prevent hydrolysis due to water in the reaction solution.

The duration of the reaction is preferably a period of time taken until the haloalkyl compound used as a raw material is consumed, and is more preferably 1 to 12 hours for stability of the haloalkyl compound and process efficiency. The reaction may be performed in water, but it may be performed in a solvent mixture of water with an alcohol such as methanol, ethanol, isopropanol, or butanol, a glycol ether such as ethylene glycol dimethyl ether, or other solvent. The reaction may also be performed by dispersing powder of the dried partially degraded starch in a hydrophilic solvent, for example, an alcohol such as methanol, ethanol, isopropanol, or butanol, or a glycol ether such as ethylene glycol dimethyl ether. When a solvent mixture is used, the percentage of the solvent other than water is preferably 50% by volume or less based on the solvent mixture. The reactor used may be a reaction vessel or an extruder, for example.

When the acidic group-containing compound used is an acid anhydride, the reaction is allowed to proceed by only mixing and heating the partially degraded starch and the acid anhydride. However, in order to promote the reaction, a catalyst may be used, such as sodium carbonate, sodium hydroxide, a tertiary amine such as triethylamine, an imidazole such as 2-methylimidazole, a quaternary ammonium salt such as tetrabutylammonium bromide, or a phosphonium salt such as tetrabutylphosphonium bromide. The amount of the catalyst to be added is preferably 0.1 equivalents or less relative to the acidic group-containing compound. These catalysts may be used alone or in combinations of two or more.

The duration of the reaction is preferably a period of time taken until the acid anhydride used is consumed, more preferably 1 to 12 hours. The end point of the reaction can be determined by the measurement of the acid value or IR measurement. The reaction may be performed in water, but the reaction solvent used is preferably an aprotic solvent such as dimethyl sulfoxide, dimethylformamide, dimethylacetamide, or N-methylpyrrolidone, in order to prevent hydrolysis or alcoholysis of the acid anhydride. When a solvent mixture is used, the percentage of the solvent other than water is preferably 50% by volume or more based on the solvent mixture. When the reaction is performed with no solvent, the reaction is preferably performed at a temperature equal to or more than the melting point of the acid anhydride because the acid anhydride can serve as a solvent. When the reaction is performed with a solvent, the reaction temperature is preferably 50°C to 100°C, more preferably 70°C to 90°C. The reactor used may be a reaction vessel or an extruder, for example.

<<Physical properties of water-soluble polymer>>

The molecular weight of the water-soluble polymer produced in the step (a2) is not limited, but the water-soluble polymer preferably has a weight average molecular weight of 500,000 to 40,000,000, more preferably 700,000 to 35,000,000 as determined by aqueous size exclusion chromatography using pullulan standards. If the weight average molecular weight is less than 500,000, the water retention properties of the water-absorbing resin tend to decrease, whereas if it is more than 40,000,000, the water absorption performance of the water-absorbing resin tends to decrease. Here, the weight average molecular weight determined by aqueous size exclusion chromatography using pullulan standards can be determined by aqueous size exclusion chromatography relative to a calibration curve of molecular weight vs. elution time prepared using pullulans with known molecular weights.

The total acid value of the water-soluble polymer is preferably 50 to 350 mg KOH/g, more preferably 70 to 300 mg KOH/g. The total acid value represents the total amount of acidic groups introduced into the water-soluble polymer. If the total acid value is less than 50 mg KOH/g or more than 350 mg KOH/g, the water-absorbing resin obtained after cross-linking the water-soluble polymer tends to have lower water absorption performance for an aqueous solution containing an electrolyte such as physiological saline.

When the acidic group on the water-soluble polymer has been introduced by reacting a carboxyl group-containing haloalkyl compound with the hydroxyl group of a starch or a partially degraded product thereof, the amount of acidic groups (carboxyalkyl groups) introduced can also be represented by the degree of etherification. The degree of etherification of the water-soluble polymer is preferably 0.1 to 2.0, more preferably 0.2 to 1.5. The degree of etherification can be determined by, for example, an ashing-titration method. Moreover, since the total acid value is detected via the introduction of a carboxyalkyl group, when the partially degraded starch used as a raw material contains no acidic group such as a carboxyalkyl group, the acidic groups detected by the measurement of the total acid value are considered to be equivalent to those introduced by an etherification reaction. Therefore, when the starch or partially degraded starch as a raw material contains no acidic group, the degree of etherification may be calculated conveniently from the total acid value. For example, when carboxymethyl groups as carboxyalkyl groups all have been neutralized into sodium salts, the degree of etherification can be calculated as follows:

$$\text{Degree of etherification} = (162 \times \text{total acid value}) \div (56100 - 80 \times \text{total acid value}).$$

Here, the total acid value in this case is expressed in the unit of mg KOH/g.

The free acid value of the water-soluble polymer is not limited, but it is preferably 0 to 70 mg KOH/g, more preferably 0 to 50 mg KOH/g. When the acidic group on the water-soluble polymer forms a salt with sodium, potassium, lithium, ammonia, or the like and the method does not include partially neutralizing the acidic group, the free acid value is preferably 0 mg KOH/g. When the method includes, after introducing an acidic group into the partially degraded starch, a step (ab) of partially neutralizing the acidic group, the free acid value is preferably 5 to 70 mg KOH/g, more preferably 7 to 50 mg KOH/g. The term "free acid value" refers to the acid value measured for acidic groups which have not been neutralized. If the free acid value is more than 70 mg KOH/g, the water absorption performance tends to decrease.

The dispersity (weight average molecular weight/number average molecular weight) of the water-soluble polymer is not limited, but it is preferably 5 to 50, more preferably 7 to 45. If the dispersity is less than 5 or more than 50, the water absorption performance of the water-absorbing resin tends to decrease. The number average molecular weight of the water-soluble polymer can be determined by aqueous size exclusion chromatography.

The water-soluble polymer preferably has a biomass content of 50% or higher, more preferably 60% or higher. The term "biomass content" refers to the percentage (% by mass) of elements derived from natural resources among the elements constituting the water-soluble polymer. The biomass content can be measured by the method described in

EXAMPLES.

<Desalting step (aa)>

The method may include, after the step (a2), (aa) desalting the water-soluble polymer. The desalting can remove the halogenated alkali metal salt generated as a byproduct in the step (a2), the salt of the unreacted haloalkyl compound used in the step (a2), and the salt generated in the partial neutralization step (ab) described later. Examples of the desalting method include desalting using a solvent and desalting using an ultrafiltration membrane. The desalting using a solvent may be performed by, for example, a washing process including dropping an aqueous solution in which the water-soluble polymer is dissolved in water into a hydrophilic solvent such as methanol, ethanol, isopropanol, acetone, or acetonitrile, reprecipitating the water-soluble polymer, and collecting it by filtration, followed by redispersing and stirring the water-soluble polymer collected by filtration in hydrous methanol (with a water content of about 70 to 90%), and then collecting the particles of the water-soluble polymer by filtration. The method using an ultrafiltration membrane may include treating an aqueous solution of the hydrophilic polymer with a filter having an ultrafiltration membrane. The washing liquid used for desalting may be water or a liquid mixture of water and a hydrophilic organic solvent such as methanol, ethanol, propanol, acetone, or acetonitrile. The desalting is preferably performed until the salt concentration in the water-soluble polymer reaches 1% or less.

<Step (ab) of partially neutralizing water-soluble polymer>

The method may include, in the step (a2) or after the step (a2) but before the step (a3), a step (ab) of partially neutralizing the acidic group in the water-soluble polymer.

In the case where the carboxyl group, sulfo group, or phospho group moiety of the acidic group introduced in the partially degraded starch forms a salt with sodium, potassium, lithium, ammonia, or the like derived from the alkaline agent in the water-soluble polymer obtained in the step (a2), the salt is neutralized with an acid in the step (ab). The neutralization converts some of the salt-forming carboxyl group, sulfo group, or phospho groups into the free carboxylic acid, sulfonic acid, or phosphoric acid form. Also, in the case where the carboxyl, sulfo, or phospho group moiety of the acidic group introduced in the partially degraded starch is in the form of non-salt forming free acid in the water-soluble polymer obtained in the step (a2), the salt is neutralized with an alkali in the step (ab). The neutralization converts some of the free carboxylic acid, sulfonic acid, or phosphoric acid groups into salt-forming carboxyl, sulfo, or phospho groups.

The partial neutralization is preferably performed on some of the salt-forming carboxyl group, sulfo group, or phospho groups on the water-soluble polymer. For example, when the acidic group-containing compound used is monochloroacetic acid and the alkaline agent used is sodium hydroxide in the step (a2), a water-soluble polymer is obtained in which a sodium salt of a carboxyl group has been added to the partially degraded starch. Adding an acid thereto converts some carboxyl groups into the free carboxylic acid form.

Moreover, the partial neutralization is preferably performed on some of the non-salt forming carboxyl group, sulfo group, or phospho groups on the water-soluble polymer. For example, when the acidic group-containing compound used in the step (a2) is succinic anhydride, a water-soluble polymer is obtained in which a free carboxylic acid has been added to the partially degraded starch. Adding an alkali thereto converts some of the free carboxylic acid moieties into salt-forming carboxy groups.

In the step (ab), the degree of neutralization of the acidic group in the water-soluble polymer after the partial

neutralization is preferably 1 to 50%, more preferably 5 to 25%. A degree of neutralization within the above range tends to facilitate the production of the water-absorbing resin. The degree of neutralization refers to the percentage of neutralized acidic groups among the acidic groups in the water-soluble polymer and is calculated by the following equation.

Degree of neutralization (%) = ((Free acid value of water-soluble polymer) $\div$ (Total acid value of water-soluble polymer)) $\times$ 100

The total acid value of the water-soluble polymer and the free acid value of the water-soluble polymer are as described above concerning the physical properties of the water-soluble polymer.

The salt-forming carboxyl group can be neutralized with an acid. The acid is not limited, but it is preferably an acid having a pKa equal to or less than that of the carboxyl group. Examples of such acids include hydrochloric acid, sulfuric acid, phosphoric acid, nitric acid, acetic acid, formic acid, and trichloroacetic acid. A strong acid is preferably used to neutralize the salt-forming sulfo group or phospho group. It is more preferred to use a mineral acid such as hydrochloric acid or sulfuric acid or a strongly acidic ion exchange resin. The neutralization can be performed using a known apparatus such as a reaction vessel or an extruder. After the acid is added, stirring is preferably performed at 0°C to 50°C for 0.2 to 1 hour for the neutralization reaction. The neutralization reaction is preferably performed at pH 6.8 to 7.2.

The free carboxylic acid, sulfonic acid, or phosphoric acid is neutralized with an alkali. Nonlimiting examples of the alkali include sodium hydroxide, potassium hydroxide, sodium carbonate, potassium carbonate, ammonia, dimethylamine, and tetramerylammonium hydroxide. The neutralization can be performed using a known apparatus such as a reaction vessel or an extruder. After the alkali is added, stirring is preferably performed at 0°C to 50°C for 0.2 to 1 hour for the neutralization reaction. The neutralization reaction is preferably performed at pH 6.8 to 7.2.

In the neutralization reaction of the salt-forming carboxyl group, sulfo group, or phospho group, a salt may be formed between the halogen derived from the acidic group-containing compound and the metal or ammonium derived from the alkaline agent. This salt can be removed by the desalting step (aa) described above.

When the step (ab) is performed in the step (a2), partial neutralization is preferably performed after the reaction between the partially degraded starch and the acidic group-containing compound in the step (a2).

However, the step (ab) is not essential. One embodiment of the present invention may be a method for producing a water-absorbing resin which does not include, after the step (a2) but before the drying step (a3), partially neutralizing the acidic group in the water-soluble polymer. Without the partial neutralization step, the cost for partial neutralization can be saved.

<Drying step (a3)>

In this step, the water-soluble polymer is dried at 70°C to 180°C in the presence of water so that it has a solid content of 90% or more. The water-soluble polymer at the beginning of the step (a3) may be in the form of an aqueous solution or water-containing wet powder.

The liquid component contained in the water-soluble polymer may be water or a solvent mixture of water and a hydrophilic solvent. Examples of the hydrophilic solvent include lower aliphatic alcohols such as methanol, ethanol, n-propanol, and isopropanol; ketones such as acetone; ethers such as dioxane, tetrahydrofuran, and methoxy(poly)ethylene glycol; and amides such as $\varepsilon$-caprolactam and N,N-dimethylformamide. Two or more of these hydrophilic solvents may be used in combination. The percentage of the hydrophilic solvent in the solvent mixture is preferably controlled depending on the boiling point of the hydrophilic solvent. When the boiling point of the hydrophilic solvent is 100°C or lower, the percentage thereof is preferably 70% by volume or higher, while the boiling point of the hydrophilic solvent is higher than 100°C, the percentage thereof is preferably 30% by volume or lower.

When the water-soluble polymer at the beginning of the step (a3) is in the form of wet powder, the liquid component content is preferably 50 to 90% by weight, more preferably 60 to 80% by weight, still more preferably 65 to 75% by weight.

The drying is performed until the solid content of the water-soluble polymer reaches 90% or more, preferably 93% or more, more preferably 95% or more, still more preferably 97% or more. The upper limit of the solid content is preferably 99% or less. Here, the solid content refers to the ratio of the weight of components other than water and hydrophilic solvents to the weight of the water-soluble polymer. By drying the water-soluble polymer so that it has a solid content of 90% or more, it is possible to produce a water-absorbing resin that forms a physical gel upon absorption of water, even when the method does not include, before the step (a3), partially neutralizing the acidic group in the water-soluble polymer.

The drying temperature is preferably 70°C to 180°C. Further, the drying temperature is more preferably selected depending on the presence or absence of the step (ab) of partially neutralizing the acidic group. When the method includes the step (ab) of partially neutralizing the acidic group, the drying temperature is preferably 70°C to 150°C, more preferably 70°C to 130°C. When the method does not include the step (ab) of partially neutralizing the acidic group, the drying

temperature is preferably 80°C to 150°C, more preferably 100°C to 130°C. The device used for drying is not limited and may be a drum dryer, a spray dryer, or a Nauta mixer, for example.

In the step (a3), a cross-linked structure is formed between one water-soluble polymer and another. The cross-links formed here correspond to the internal cross-links of the water-absorbing resin. The cross-links are preferably formed via the acidic group introduced onto the water-soluble polymer in the step (a2). Examples of the cross-linked structure include an ionic bond between the acidic groups, a coordination bond via a metal ion, and a hydrogen bond resulting from the dimerization of the acidic groups. The cross-links may include covalent cross-links as long as they do not inhibit degradation of the resulting water-absorbing resin. The percentage of covalent cross-links, if present, is preferably 5% or less, more preferably 2% or less of the cross-linked structure. Examples of the covalent cross-links include an ester bond, an ether bond, a carbon-carbon single bond (C-C bond), and a carbon-carbon double bond (C=C bond).

Although the cross-linked structure can be formed without using a cross-linking agent as described above, it may be formed using a cross-linking agent. Examples of the cross-linking agent include epoxy compounds, polyhydric alcohol compounds, polyamine compounds, polyisocyanate compounds, alkylene carbonate compounds, haloepoxy compounds, halohydrin compounds, polyvalent oxazoline compounds, carbodiimide compounds, silane coupling agents, and polyvalent metal compounds.

Examples of the epoxy compounds include glycidyl succinate, sorbitol polyglycidyl ether, trimethylolpropane polyglycidyl ether, ethylene glycol diglycidyl ether, polyethylene glycol diglycidyl ether, glycerol polyglycidyl ether, diglycerol polyglycidyl ether, polyglycerol polyglycidyl ether, propylene glycol diglycidyl ether, polypropylene glycol diglycidyl ether, and glycidol.

Examples of the polyhydric alcohol compounds include ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerol, polyglycerol, 2-butene-1,4-diol, 1,3-butanediol, 1,4-butanediol, 1,5-pentanediol, 1,6-hexanediol, 1,2-cyclohexanedimethanol, 1,2-cyclohexanediol, trimethylolpropane, diethanolamine, triethanolamine, polyoxypropylene, oxyethylene-oxypropylene block copolymers, pentaerythritol, and sorbitol.

Examples of the polyamine compounds include ethylenediamine, diethylenetriamine, triethylenetetramine, tetraethylenepentamine, pentaethylenehexamine, polyethyleneimine, inorganic or organic salts of these polyamine compounds (such as azitinium salts), and polysaccharides having amino groups such as chitin.

Examples of the polyisocyanate compounds include 2,4-tolylene diisocyanate and hexamethylene diisocyanate, and examples of the polyvalent oxazoline compounds include 1,2-ethylenebisoxazoline.

Examples of the alkylene carbonate compounds include 1,3-dioxolan-2-one, 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, and 4,6-dimethyl-1,3-dioxan-2-one.

Examples of the haloepoxy compounds include epichlorohydrin, epibromohydrin, α-methylepichlorohydrin, and polyamine adducts thereof (for example, Kymene® available from Hercules Incorporated).

Other examples of known cross-linking agents that can be used include waterborne carbodiimide compounds (for example, CARBODILITE available from Nisshinbo Chemical Inc.); silane coupling agents such as γ-glycidoxypropyltrimethoxysilane and γ-aminopropyltriethoxysilane; and polyvalent metal compounds such as hydroxides or chlorides of zinc, calcium, magnesium, aluminum, iron, zirconium, etc.

In the step (a3), a constitutional unit other than the water-soluble polymer may optionally be incorporated and cross-linked. Examples of the constitutional unit other than the water-soluble polymer include polyacrylic acids (or salts thereof) such as cross-linked products of partially neutralized polyacrylic acids, self-cross-linking partially neutralized polyacrylic acids, and starch-acrylic acid graft polymers. Examples of the salts of acrylic acids include sodium salts, potassium salts, and ammonium salts. Other examples of the constitutional unit other than the water-soluble polymer include anionic unsaturated monomers and salts thereof such as methacrylic acid, maleic acid, vinylsulfonic acid, styrenesulfonic acid, 2-(meth)acrylamido-2-methylpropanesulfonic acid, 2-(meth)acryloylethanesulfonic acid, and 2-(meth)acryloylpropanesulfonic acid; hydrophilic group-containing nonionic unsaturated monomers such as acrylamide, methacrylamide, N-ethyl(meth)acrylamide, N-n-propyl(meth)acrylamide, N-isopropyl(meth)acrylamide, N,N-dimethyl(meth)acrylamide, 2-hydroxyethyl (meth)acrylate, 2-hydroxypropyl (meth)acrylate, methoxypolyethylene glycol (meth)acrylate, polyethylene glycol mono(meth)acrylate, vinylpyridine, N-vinylpyrrolidone, N-acryloylpiperidine, and N-acryloylpyrrolidine; cationic unsaturated monomers such as N,N-dimethylaminoethyl (meth)acrylate, N,N-diethylaminoethyl (meth)acrylate, N,N-dimethylaminopropyl (meth)acrylate, N,N-dimethylaminopropyl(meth)acrylamide, and quaternary salts thereof; linear celluloses, and poly (γ-glutamic acid) .

When the water-absorbing resin contains a constitutional unit other than the water-soluble polymer, the amount of the constitutional unit is preferably 90% by weight or less, more preferably 50% by weight or less, still more preferably 20% by weight or less, relative to the combined amount of the constitutional unit and the water-soluble polymer used as a main component.

The water-absorbing resin may contain other additives, such as a disinfectant, a deodorant, an antimicrobial agent, a fragrance, various inorganic powders, a foaming agent, a pigment, a dye, a hydrophilic short fiber, a fertilizer, an

oxidizing agent, a reducing agent, water, and salts, in order to impart various functions. The method of adding these additives and the amounts of these additives can be appropriately selected by those skilled in the art.

Surface cross-linking may be performed after the drying step (a3). The surface cross-linking can improve the strength of the water-absorbing resin. Examples of cross-linking agents that may be used for surface cross-linking include the cross-linking agents described above for the cross-linking in the step (a3). Preferred among these are epoxy compounds, with ethylene glycol diglycidyl ether, sorbitol polyglycidyl ether, or glycidyl succinate being more preferred.

Surface cross-links can be formed by spraying a surface cross-linking agent to the water-absorbing resin, and then mixing them by a known method with a cylindrical mixer, a V-shaped mixer, a ribbon mixer, a screw mixer, a twin-arm mixer, a pulverizing kneader, or the like, to cause cross-linking. A surfactant may be added as necessary during the spraying and mixing.

The produced water-absorbing resin may be subjected to a treatment such as desalting or washing to remove impurities and by-products. Desalting can be performed with a filter having a reverse osmosis membrane, for example. The washing liquid used may be water or a liquid mixture of water and a hydrophilic organic solvent such as methanol, ethanol, or propanol.

<<Method 2 for producing water-absorbing resin>>

The production method of the present invention includes: (b1) introducing an acidic group into a starch; (b2) reducing the molecular weight of the acidic group-containing starch to obtain a water-soluble polymer; and (b3) drying the water-soluble polymer at 70°C to 180°C in the presence of water so that it has a solid content of 90% or more.

<Step (b1) of introducing acidic group into starch>

In this step, a starch is reacted with an acidic group-containing compound. The starch, the acidic group, and the acidic group-containing compound used are as described above concerning the step (a2) of introducing an acidic group into the partially degraded starch.

The conditions of the reaction between the starch and the acidic group-containing compound are not limited. When the acidic group-containing compound used is a carboxyl group-containing haloalkyl compound, an alkaline agent is preferably used in an amount of 1 to 1.5 equivalents relative to the acidic group-containing compound. The pH is preferably 10.5 to 12.5, more preferably 11 to 12, to stabilize the starch. Examples of the alkaline agent used for pH adjustment include sodium hydroxide, potassium hydroxide, lithium hydroxide, ammonia, sodium carbonate, and potassium carbonate.

The acidic group that has been introduced into the starch preferably forms a salt with sodium, potassium, lithium, ammonia, or the like derived from the alkaline agent. Therefore, the alkaline agent is preferably used in an amount required for both the reaction between the acidic group-containing compound and the starch and the neutralization of the acidic group of the acidic group-containing compound. For example, when the acidic group-containing compound used is monochloroacetic acid, the alkaline agent is preferably used theoretically in an amount of at least two equivalents relative to the monochloroacetic acid. When sodium monochloroacetate is used, the alkaline agent is preferably used in an amount of at least one equivalent relative to the sodium chloroacetate because the acidic group has already been neutralized.

The amount of the acidic group-containing compound to be used can be arbitrarily set depending on the desired total acid value (degree of etherification) of the water-soluble polymer. Usually, the amount is preferably 0.5 to 5.0 equivalents, more preferably 0.5 to 2.0 equivalents per mole of hydroxyl groups of the starch. When a haloalkyl compound such as monochloroacetic acid in the form of an aqueous solution is to be reacted, the haloalkyl compound is required in excess of the theoretical amount because the introduction reaction of the acidic group will compete with the hydrolysis reaction of the haloalkyl compound. The amount of the haloalkyl compound to be used in the reaction of the aqueous solution is preferably set to be 5 equivalents or less relative to the theoretical value.

The temperature during the reaction between the starch and the acidic group-containing compound is not limited, but it is preferably 0°C to 120°C, more preferably 0°C to 100°C. The duration of the reaction is preferably 1 to 24 hours, though not limited thereto. The reaction may be performed in water, but it may be performed in a solvent mixture of water with an alcohol such as methanol, ethanol, isopropanol, butanol, ethylene glycol, diethylene glycol, propylene glycol, or ethylene glycol monoethyl ether, a glycol ether such as ethylene glycol dimethyl ether, or other solvent. When a solvent mixture is used, the percentage of the solvent other than water is preferably 50% by volume or less based on the solvent mixture. The reactor used may be a reaction vessel or an extruder, for example.

In particular, when the haloalkyl compound used is chloroacetic acid or a salt thereof, the reaction is preferably performed at 25°C to 60°C to prevent hydrolysis due to water in the reaction solution. The duration of the reaction is preferably a period of time taken until the haloalkyl compound used as a raw material is consumed, and is more preferably one to six hours for stability of the haloalkyl compound and process efficiency. The reaction may be performed in water., but it may be performed in a solvent mixture of water with an alcohol such as methanol, ethanol, isopropanol, or butanol, a glycol ether such as ethylene glycol dimethyl ether, or other solvent. The reaction may also be performed by dispersing

dried starch powder in a hydrophilic solvent, for example, an alcohol such as methanol, ethanol, isopropanol, or butanol, or a glycol ether such as ethylene glycol dimethyl ether. When a solvent mixture is used, the percentage of the solvent other than water is preferably 50% by volume or less based on the solvent mixture. The reactor used may be a reaction vessel or an extruder, for example.

Also, when the acidic group-containing compound used is an acid anhydride, the reaction is allowed to proceed by only mixing and heating the starch and the acid anhydride. However, in order to promote the reaction, a catalyst may be used, such as sodium carbonate, sodium hydroxide, a tertiary amine such as triethylamine, an imidazole such as 2-methylimidazole, a quaternary ammonium salt such as tetrabutylammonium bromide, or a phosphonium salt such as tetrabutylphosphonium bromide. The amount of the catalyst to be added is preferably 0.1 equivalents or less relative to the acidic group-containing compound. These catalysts may be used alone or in combinations of two or more.

The duration of the reaction is preferably a period of time taken until the acid anhydride as a raw material is consumed, more preferably 1 to 12 hours. The end point of the reaction can be determined by the measurement of the acid value or IR measurement. The reaction may be performed in water, but the reaction solvent used is preferably an aprotic solvent such as dimethyl sulfoxide, dimethylformamide, dimethylacetamide, or N-methylpyrrolidone, in order to prevent hydrolysis or alcoholysis of the acid anhydride. When a solvent mixture is used, the percentage of the solvent other than water is preferably 50% by volume or more based on the solvent mixture. When the reaction is performed with no solvent, the reaction is preferably performed at a temperature equal to or more than the melting point of the acid anhydride because the acid anhydride can serve as a solvent. When the reaction is performed with a solvent, the reaction temperature is preferably 50°C to 100°C, more preferably 70°C to 90°C. The reactor used may be a reaction vessel or an extruder, for example.

<Step (b2) of obtaining water-soluble polymer>

In this step, the molecular weight of the acidic group-containing starch obtained in the step (b1) is reduced to decrease the viscosity. The molecular weight of the starch may be reduced by any method, such as by subjecting the starch to enzymatic treatment, acid treatment, physical crushing, or other method. These methods are as described above concerning the step (a1) of reducing the molecular weight of a starch to obtain a partially degraded starch.

After the step (a2), the water-soluble polymer may be desalted as in the step (aa) described above. Moreover, the acidic group in the water-soluble polymer may be partially neutralized as in the step (ab) described above.

<Drying step (b3)>

In this step, the water-soluble polymer is dried at 70°C to 180°C in the presence of water so that it has a solid content of 90% or more. The water-soluble polymer at the beginning of the step (b3) may be in the form of an aqueous solution or water-containing wet powder. The liquid component and its amount in the water-soluble polymer, the drying conditions, the solid content, and the cross-linked structure formed between one water-soluble polymer and another used are as described above concerning the drying step (a3).

<<Cross-linked structure of water-absorbing resin>>

Generally, water-absorbing resins containing a polyacrylic acid as a main constitutional unit have a covalent network structure and form a chemical gel upon absorption of water. In contrast, the water-absorbing resin obtained by the method 1 for producing a water-absorbing resin or method 2 for producing a water-absorbing resin of the present invention is characterized by forming a physical gel upon absorption of water. The cross-linking points in the physical gel may relatively easily disappear due to, for example, the thermal motion of the molecular chains or changes in pH or ionic strength, so that the physical gel can be converted into flowable sol.
Therefore, the physical gel can be easily degraded by, for example, adding an alkali or an acid, heating, or shaking, which leads to a reduction of the environmental burden at the time of disposal.

The formation of a physical gel can be confirmed by analyzing the sol-gel transition based on the disappearance of cross-linking points. For example, it may be confirmed by fluidization after alkali or acid treatment when the gel has been cross-linked via ionic bonds and/or hydrogen bonds. Specifically, when a water-absorbing resin is suspended in a 1% aqueous sodium hydroxide solution to a final concentration of 5% by weight, stirred for 60 minutes, and then sieved through a sieve having an opening of 500 μm, the water-absorbing resin can be considered to form a physical gel upon absorption of water if the dry weight of the water-absorbing resin remaining on the sieve is less than 2% by weight of the dry weight of the water-absorbing resin contained in the aqueous solution.

More specifically, 0.5 g of a water-absorbing resin may be suspended in 9.5 g of a 1% aqueous NaOH solution, stirred for 60 minutes, and then subjected to natural filtration through an about 50 mm × 50 mm square 30-mesh sieve (opening: 500 μm), followed by washing the top of the sieve with ion-exchanged water. After the washing, the sieve may be

dried with a fan dryer at 120°C for two hours. The water-absorbing resin can be considered to form a physical gel upon absorption of water if the 30-mesh pass residue calculated by the following equation is less than 2% by weight, in which Wm1 represents the weight of the sieve before filtration and Wm2 represents the weight of the sieve after filtration and drying. Here, the sieve having an opening of 500 pm corresponds to a 30-mesh sieve stipulated in JIS.

$$\text{30-Mesh pass residue (\%)} = (Wm2 - Wm1)/0.5 \times 100$$

The 30-mesh pass residue is preferably less than 2% by weight, more preferably less than 1.5% by weight, still more preferably less than 1.0% by weight, further preferably less than 0.5% by weight.

The formation of a physical gel upon absorption of water may also be confirmed by observing the properties of the water-absorbing resin after adding water thereto. Specifically, 0.2 g of a water-absorbing resin may be put into a nylon mesh tea bag and then immersed and left in 1 L of water for three hours. Then, the solubility in water and the gel strength may be evaluated based on the criteria described in EXAMPLES. With an evaluation score of 2 to 6, a physical gel can be considered to be formed.

<<Water absorption performance of water-absorbing resin>>

The water absorption rate under no pressure of the water-absorbing resin obtained by the method 1 for producing a water-absorbing resin or method 2 for producing a water-absorbing resin of the present invention is determined by measuring the absorbency for physiological saline or ion-exchanged water of the water-absorbing resin with no load applied thereto as described in EXAMPLES. The water-absorbing resin in a solid state preferably has a water absorption rate under no pressure for ion-exchanged water of 100 to 500 g/g, more preferably 120 to 460 g/g. Also, the water absorption rate under no pressure for physiological saline is preferably 10 to 70 g/g, more preferably 20 to 70 g/g, still more preferably 30 to 65 g/g.

The water-absorbing resin preferably has a ratio (A/B) of the water absorption rate under no pressure for ion-exchanged water (A) to the water absorption rate under no pressure for physiological saline (B) of 7 or less, more preferably 5 or less.

The water retention rate of the water-absorbing resin is determined by measuring the absorbency for physiological saline or ion-exchanged water of the water-absorbing resin with a load of 150 G applied thereto as described in EXAMPLES. The water-absorbing resin in a solid state preferably has a water retention rate for ion-exchanged water of 80 to 300 g/g, more preferably 100 to 300 g/g. Also, the water retention rate for physiological saline is preferably 5 to 65 g/g, more preferably 10 to 60 g/g, still more preferably 20 to 60 g/g.

The biomass content of the water-absorbing resin is preferably 50% or more, more preferably 60% or more. The term "biomass content" refers to the percentage (% by mass) of elements derived from natural resources among the elements constituting the water-soluble resin. For example, the biomass content can be calculated from the biomass content of the water-soluble polymer measured as described in EXAMPLES, taking into account molecular weight variations due to partial neutralization or cross-linking.

<Method for degrading water-absorbing resin>

The water-absorbing resin obtained by the method 1 for producing a water-absorbing resin or method 2 for producing a water-absorbing resin of the present invention may be degraded by alkali treatment. In the alkali treatment, the water-absorbing resin is preferably placed at pH 9 or higher, more preferably pH 10 or higher. The alkali treatment can cleave the cross-linked structure and glucoside bonds of the water-absorbing resin to degrade the water-absorbing resin into water-soluble polymers, thereby reducing the environmental burden at the time of disposal.

Examples of the alkaline agent used in the alkali treatment include, but are not limited to, sodium hydroxide, potassium hydroxide, lithium hydroxide, ammonia, sodium carbonate, and potassium carbonate. The alkali treatment can be performed at any temperature, for example, at 5°C to 50°C.

<<Article containing water-absorbing resin>>

Various articles can be produced from the water-absorbing resin obtained by the method 1 for producing a water-absorbing resin or method 2 for producing a water-absorbing resin of the present invention. Examples of the articles include hygiene and medical products such as paper diapers, sanitary products, incontinence pads, portable toilets, disposal bags, animal waste disposal agents, medical treatment materials, surgical sheets, dental waste disposal agents, medical waste blood coagulating agents, wound dressings, and moisturizing agents; agricultural and gardening products such as soil water retention agents, seedling sheets, seed coatings, sustained release agents for fertilizers, disintegration

aids for agricultural chemicals and fertilizers, desert greening materials, agricultural films, and freshness-keeping agents; civil engineering materials such as soil modifiers, sludge solidifying agents, and water blocking materials; as well as articles such as refrigerants, thickening agents, deodorants, drip absorbing agents for food, pet sheets, disposable body warmers, gelling agents for batteries, dew condensation preventing sheets, packing materials, and artificial snow. Examples of the hygiene products include laminates having a back sheet, an absorber, and a top sheet stacked in this order. The absorber contains the water-absorbing resin of the present invention and may further contain a water-absorbing paper or pulp, as necessary. The specific methods for producing these articles are not limited, and each article can be produced by a well-known method.

These articles containing the water-absorbing resin described above are characterized by being easily degradable. The water-absorbing resin can be degraded under alkali treatment conditions, thereby reducing the environmental burden at the time of disposal.

EXAMPLES

Hereinafter, the present invention will be described in detail with reference to examples, but the present invention is not limited thereto. Hereinafter, "part(s)" and "%" mean "part(s) by weight" and "% by weight" respectively, unless otherwise specified.

(1) Materials used

(1-1) Starch raw material

Corn starch
Tapioca starch
Tapioca starch acetate
Acetylated oxidized tapioca starch
Acetylated tapioca starch
Hydroxypropyl starch
Hydroxypropyl distarch phosphate

(1-2) Hydrolase
$\alpha$-amylase (Spitase® HK/R available from Nagase ChemteX Corporation), 12,200 units/g

Amylomaltase: *Thermus thermophilus* was aerobically cultured. The homogenate extract of the collected bacterial cells was centrifuged, and the supernatant was used as a crude enzyme solution. The crude enzyme solution was subjected to column chromatography in a conventional manner and then electrophoretically purified to a single product, and this sample was used as a purified enzyme solution.

Here, the activity of amylomaltase was determined in the following manner. A reaction liquid 1 containing 10 w/v % maltotriose, a 50 mM sodium acetate buffer (pH 6.0), and the enzyme was incubated at 60°C for 20 minutes. Thereafter, the reaction was stopped by heating at 100°C for 10 minutes. The amount of glucose in the reaction liquid was measured by a glucose oxidase method. For the unit amount of amylomaltase, the activity of amylomaltase that produces 1 pmol of glucose per minute was defined as one unit.

(2) Production of partially degraded starch (Production Examples 1 to 7)

Partially degraded starches were produced in the following manner. Here, the weight average molecular weight of each partially degraded starch obtained was determined by aqueous size exclusion chromatography relative to a calibration curve of molecular weight vs. elution time prepared using pullulans with known molecular weights. Table 1 shows the weight average molecular weight and dispersity of each partially degraded starch.

(Production Example 1) Partially degraded starch derived from corn starch

Corn starch was suspended in city water to a concentration of 15% (w/w), and then 1N sodium hydroxide was added thereto to adjust the pH to 6.0 to obtain a starch milk. To the starch milk were added 0.4 units of the crude enzyme solution of amylomaltase per gram of the starch solids. The mixture was stirred at room temperature for 30 minutes and then reacted at 80°C for six hours while stirring to prepare a liquefied starch. The obtained partially degraded starch had a

weight average molecular weight of 1,930,000 and a dispersity of 9.6.

(Production Example 2)

Corn starch was suspended in city water to a concentration of 30% (w/w), and then 1N sodium hydroxide was added thereto to adjust the pH to 6.0 to obtain a starch milk. To the starch milk were added 0.15 units of the crude enzyme solution of amylomaltase per gram of the starch solids and 0.04 units of α-amylase per gram of the starch solids. The mixture was stirred at room temperature for 30 minutes and then reacted at 80°C for six hours while stirring to prepare a liquefied starch. The obtained partially degraded starch had a weight average molecular weight of 1,340,000 and a dispersity of 10.6.

(Production Example 3)

A liquefied starch was prepared by the same procedure as in Production Example 1, except that the concentration of the corn starch was changed to 30% (w/w) and 0.2 units of the crude enzyme solution of amylomaltase per gram of the starch solids was used. The obtained partially degraded starch had a weight average molecular weight of 1,970,000 and a dispersity of 10.5.

(Production Example 4)

A liquefied starch was prepared by the same procedure as in Production Example 3, except that a corn starch from a different lot was used. The obtained partially degraded starch had a weight average molecular weight of 1,910,000 and a dispersity of 10.1.

(Production Example 5)

A liquefied starch was prepared by the same procedure as in Production Example 3, except that the production was scaled up 14-fold while maintaining the concentrations of the corn starch and the enzyme. The obtained partially degraded starch had a weight average molecular weight of 5,160,000 and a dispersity of 26.3. The molecular weight and the dispersity were increased compared to those of Production Example 3 probably because the reaction efficiency changed due to the scale-up.

(Production Example 6)

The aqueous solution of the partially degraded starch obtained in Production Example 2 was dried in vacuum at 80°C. The resulting dried product was pulverized to collect a dry powder product of 1 mm pass. The moisture content of the dry powder product was 2.7%. Here, the molecular weight and dispersity of the obtained partially degraded starch were as described in Production Example 2.

(Production Example 7)

Corn starch was suspended in city water to a concentration of 15% (w/w), and then 1N sodium hydroxide was added thereto to adjust the pH to 6.0 to obtain a starch milk. To the starch milk were added 0.5 units of α-amylase per gram of the starch solids. The mixture was stirred at room temperature for 30 minutes and then reacted at 100°C for 20 minutes while stirring to prepare a liquefied starch. The obtained partially degraded starch had a weight average molecular weight of 1,370,000 and a dispersity of 65.6. The dispersity was increased compared to that of Production Example 1 probably because the reaction efficiency changed due to the scale-up and the use of a different enzyme, though the concentration of the corn starch was the same as in Production Example 1.

[Table 1]

| | Production Example 1 | Production Example 2, 6 | Production Example 3 | Production Example 4 | Production Example 5 | Production Example 7 |
|---|---|---|---|---|---|---|
| Mw ($\times 10^4$) | 193 | 134 | 197 | 191 | 516 | 137 |
| Dispersity | 9.6 | 10.6 | 10.5 | 10.1 | 26.3 | 65.6 |

(3) Production of water-soluble polymer

(Production Example 8)

A 200 g portion of a 15% by weight aqueous solution of the partially degraded starch produced in Production Example 1 (0.56 mol of hydroxyl groups of the partially degraded starch) was charged into a 500 ml separable flask equipped with a stirrer, a thermometer, and a condenser tube. Next, 76.5 g of a 48.8% aqueous sodium hydroxide solution (0.93 mol, 1.68 equivalents relative to the hydroxyl groups of the partially degraded starch) was charged into the flask, and stirred at 60°C or lower until the solution became completely homogeneous. After confirming that the solution was homogeneous, an aqueous solution prepared by dissolving 42.0 g of monochloroacetic acid (0.44 mol, 0.8 equivalents relative to the hydroxyl groups of the partially degraded starch) in 10.5 g of ion-exchanged water was charged dropwise at 50°C to 60°C over 30 minutes. After charging the aqueous monochloroacetic acid solution, the temperature was adjusted to 45°C to 50°C, and the mixture was stirred for 10 hours. The end point of the reaction was determined by sampling the reaction liquid and measuring the chlorine ion content in the reaction liquid by potentiometric titration with a 0.01N silver nitrate solution, and was defined as the point at which the chlorine ion content reached at least 98% of the calculated chlorine ion content of 4.8%, which corresponds to the chlorine ion content when all the monochloroacetic acid has reacted. In this production example, the chlorine content was found to be 4.8%.

After completion of the reaction, the reaction liquid was diluted with 200 g of ion-exchanged water. The diluted reaction liquid was cooled to room temperature and added to 1.7 L of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 0.9 L of hydrous methanol with a methanol/water ratio of 80/20 (by volume) and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01N silver nitrate solution, and the washing process was repeated until the chlorine content was less than 1%. The resulting water-soluble polymer had a total acid value of 166 mg KOH/g and a degree of etherification calculated from the total acid value of 0.63.

(Production Examples 9 to 14)

Water-soluble polymers were obtained by performing the reaction as in Production Example 8, except that the raw materials and the amounts thereof charged and the reaction temperature were changed as shown in Table 2.

(Production Example 15)

A 200 g portion of a 30% by weight aqueous solution of the partially degraded starch produced in Production Example 4 (1.11 mol of hydroxyl groups of the partially degraded starch) was charged into a 500 ml separable flask equipped with a stirrer, a thermometer, and a condenser tube. Next, 79.4 g of a 48.8% aqueous sodium hydroxide solution (0.96 mol, 0.86 equivalents relative to the hydroxyl groups of the partially degraded starch) was charged into the flask and then stirred at 60°C or lower until the solution became completely homogeneous. After confirming that the solution was homogeneous, 89.9 g of crystals of 6-bromohexanoic acid (0.46 mol, 0.4 equivalents relative to the hydroxyl groups of the partially degraded starch) were charged in small portions at 50°C to 60°C over 30 minutes. After charging the 6-bromohexanoic acid, the temperature was adjusted to 45°C to 50°C and the mixture was stirred for 10 hours. The end point of the reaction was determined by sampling the reaction liquid and measuring the bromide ion content in the reaction liquid by potentiometric titration with a 0.01N silver nitrate solution, and was defined as the point at which the bromide ion content reached at least 98% of the calculated bromide ion content of 9.8%, which corresponds to the bromide ion content when all the 6-bromohexanoic acid has reacted. In this production example, the bromine content was found to be 10.1%.

After completion of the reaction, the reaction liquid was diluted with 250 g of ion-exchanged water. The diluted reaction liquid was cooled to room temperature and added to 1.6 L of ethanol over about 30 minutes to reprecipitate a water-soluble polymer. After adding all the reaction liquid, the mixture was stirred for 30 minutes, and the water-soluble polymer dispersed in ethanol was subjected to solid-liquid separation by filtration under reduced pressure.

Subsequently, in order to remove the sodium bromide contained in the water-soluble polymer, the collected water-soluble polymer was redispersed in 0.5 L of hydrous ethanol with an ethanol/water ratio of 90/10 (by volume) and then stirred and washed at room temperature for 30 minutes, followed by performing solid-liquid separation by filtration under reduced pressure to collect the water-soluble polymer again. The bromine content of the collected water-soluble polymer was measured by potentiometric titration with a 0.01N silver nitrate solution, and the washing process was

repeated until the bromide ion content was less than 1%. The resulting water-soluble polymer had a total acid value of 145 mg KOH/g and a degree of etherification calculated from the total acid value of 0.64.

[Table 2]

| Water-soluble polymer | | Production Example 8 | Production Example 9 | Production Example 10 | Production Example 11 | Production Example 12 | Production Example 13 | Production Example 14 | Production Example 15 |
|---|---|---|---|---|---|---|---|---|---|
| Partially hydrolyzed starch 1 | | Production Example 1 | Production Example 1 | Production Example 1 | Production Example 2 | Production Example 3 | Production Example 4 | Production Example 5 | Production Example 4 |
| | Concentration of aqueous solution (%) | 15 | 15 | 15 | 30 | 30 | 30 | 30 | 30 |
| | Amount of aqueous solution (g) | 200.0 | 200.0 | 200.0 | 200.0 | 200.0 | 200.0 | 200.0 | 200.0 |
| Alkaline agent | 48.8% NaOH aqueous solution (g) | 76.5 | 76.5 | 33.5 | 79.4 | 79.4 | 79.4 | 79.4 | 79.4 |
| Aqueous solution containing carboxyl alkyl group-containing compound | Monochloroacetic acid (g) | 42.0 | 42.0 | 18.4 | 43.6 | 43.6 | 43.6 | 43.6 | - |
| | Monobromohexanoic acid (g) | - | - | - | - | - | - | - | 89.9 |
| | Water for dissolving monochloroacetic acid (g) | 10.5 | 10.5 | 4.6 | 10.9 | 10.9 | 10.9 | 10.9 | - |
| Reaction temperature (°C) | | 45 to 50 | 80 to 90 | 80 to 90 | 45 to 50 | 45 to 50 | 45 to 50 | 45 to 50 | 45 to 50 |
| Water for diluting reaction liquid (g) | | 200 | 200 | 150 | 300 | 500 | 300 | 300 | 250 |
| Methanol for reprecipitation (L) | | 1.7 | 1.7 | 1.5 | 1.6 | 2.9 | 1.6 | 1.6 | 1.6*1 |
| Hydrous methanol for washing (L) | | 0.9 | 0.9 | 0.7 | 1.0 | 1.0 | 0.7 | 0.7 | 0.5*2 |
| Physical properties of water-soluble polymer | Mw ($\times 10^6$) | 5.1 | 5.1 | 4.6 | 4.1 | 8.8 | 9.3 | 34.6 | 4.8 |
| | Mw/Mn | 14.1 | 150 | 14.1 | 19.3 | 21.8 | 19.5 | 49.6 | 14.0 |
| | Total acid value (mg KOH/g) | 166 | 147 | 94 | 147 | 142 | 137 | 147 | 145 |
| | Degree of etherification | 0.63 | 0.54 | 0.31 | 0.53 | 0.51 | 0.49 | 0.53 | 0.64 |
| Biomass content (% by mass) | | 76 | 79 | 87 | 79 | 80 | 80 | 79 | 65 |

*1: Ether was used.
*2: Hydrous ethanol was used instead of hydrous methanol.

(Production Example 16)

A 27.2 g portion of the powder of the partially hydrolyzed starch produced in Production Example 7 (0.47 mol of hydroxyl groups of the partially degraded starch) and 58.1 g of dimethyl sulfoxide (DMSO) were charged and dissolved in a 300 ml separable flask equipped with a stirrer, a thermometer, and a condenser tube. Next, 14.2 g of succinic anhydride (0.14 mol, 0.30 equivalents relative to the hydroxyl groups of the partially degraded starch) was charged into the flask and stirred at 70°C to 75°C for one hour for reaction. After completion of the stirring, the reaction liquid was sampled and subjected to neutralization titration with 0.1N NaOH. The reaction liquid had an acid value of 85 mg KOH/g (theoretical end point acid value: 80.1 mg KOH/g).

After completion of the reaction, the reaction solution was diluted by adding 125 g of ion-exchanged water. A 10.8 g portion (0.13 mol) of a 48% aqueous NaOH solution was further added to neutralize 93% of the theoretical amount of the carboxylic acid introduced by the reaction with succinic anhydride to give a sodium salt. Subsequently, the resulting solution was added to 750 ml of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure and then collected.

Subsequently, in order to remove the unreacted succinic anhydride contained in the water-soluble polymer and the succinic acid generated by hydrolysis, the collected water-soluble polymer was redispersed in 500 mL of methanol and then stirred and washed at room temperature for 30 minutes, followed by performing solid-liquid separation by filtration under reduced pressure to collect the water-soluble polymer again. The total acid value and free acid value of the obtained water-soluble polymer were measured, and it was confirmed that the water-soluble polymer had a total acid value of 138 mg KOH/g and a free acid value of 37 mg KOH/g and had been partially neutralized. The obtained water-soluble polymer had a weight average molecular weight of $3.7 \times 10^6$ and a dispersity of 13.8.

(Production Example 17)

A 20.0 g portion of the powder of the partially hydrolyzed starch produced in Production Example 6 (0.36 mol of hydroxyl groups of the partially degraded starch) and 110 g of dimethyl sulfoxide (DMSO) were charged and dissolved in a 300 ml separable flask equipped with a stirrer, a thermometer, and a condenser tube. Next, 10.6 g of maleic anhydride (0.11 mol, 0.30 equivalents relative to the hydroxyl groups of the partially degraded starch) was charged into the flask and stirred at 90°C to 95°C for three hours for reaction. After completion of the stirring, the reaction liquid was sampled and subjected to neutralization titration with 0.1N NaOH. The reaction liquid had an acid value of 70 mg KOH/g (theoretical end point acid value: 43 mg KOH/g).

After completion of the reaction, the reaction liquid was added to 2.5 L of acetone over about 30 minutes to reprecipitate a water-soluble polymer. After adding all the reaction liquid, the mixture was stirred for 30 minutes, and the water-soluble polymer dispersed in acetone was subjected to solid-liquid separation by filtration under reduced pressure and then collected.

Subsequently, in order to remove the unreacted maleic anhydride contained in the water-soluble polymer and the maleic acid generated by hydrolysis, the collected water-soluble polymer was redispersed in 500 mL of acetone and then stirred and washed at room temperature for 30 minutes, followed by performing solid-liquid separation by filtration under reduced pressure to collect the water-soluble polymer again. The obtained water-soluble polymer had a total acid value of 90 mg KOH/g. The obtained water-soluble polymer had a weight average molecular weight of $3.1 \times 10^6$ and a dispersity of 39.6.

[Table 3]

| Water-soluble polymer | | Production Example 16 | Production Example 17 |
|---|---|---|---|
| Partially hydrolyzed starch 1 | | Production Example 7 | Production Example 6 |
| | Powder weight (g) | 27.2 | 20 |
| DMSO (g) | | 58.1 | 110 |
| Acid anhydride (g) | Succinic anhydride | 14.2 | - |
| | Maleic anhydride | - | 10.6 |
| Reaction temperature (°C) | | 70 to 75 | 90 to 95 |
| Water for diluting reaction liquid (g) | | 125 | - |
| 48% aqueous NaOH solution (g) | | 10.8 | - |

(continued)

| Water-soluble polymer | | Production Example 16 | Production Example 17 |
|---|---|---|---|
| Solvent for reprecipitation (L) | Methanol | 0.75 | - |
| | Acetone | - | 2.5 |
| Solvent for washing (L) | Methanol | 0.5 | - |
| | Acetone | - | 0.5 |
| Physical properties of water-soluble polymer | Mw ($\times 10^6$) | 3.7 | 3.1 |
| | Mw/Mn | 13.8 | 39.6 |
| | Total acid value (mg KOH/g) | 138 | 90 |
| | Free acid value (mg KOH/g) | 37 | - |

(4) Production of water-absorbing resin

(4-1) Method not including partial neutralization

(Example 1)

A 25 g portion (wet crystals) of the water-soluble polymer obtained in Production Example 9 (product wet with hydrous methanol; wet ratio: 67%) was transferred to a petri dish, introduced into a fan dryer set at 100°C, and dried for 20 hours to perform cross-linking treatment. The solids obtained after the treatment were pulverized in a mortar and sieved through sieves with openings of 150 pm and 850 µm to collect particles having a particle size of 150 to 850 pm.

(Examples 2 to 9 and 13 and Comparative Examples 2 and 3)

Water-soluble resins were obtained by the same procedure as in Example 1, except that the raw materials and the amounts thereof charged were changed as shown in Table 4.

(4-2) Method including partial neutralization

(Example 10)

A 25 g portion of the water-soluble polymer obtained in Production Example 12 (product wet with hydrous methanol; wet ratio: 71%) was introduced into a 300 ml beaker, and 100 ml of hydrous methanol with a methanol/water ratio = 80/20 (by volume) was further added to disperse the water-soluble polymer. To this dispersion, 1.1 ml of 1N hydrochloric acid was gradually added with a measuring pipette while stirring with a magnetic stirrer. After adding the hydrochloric acid, the mixture was stirred for 15 minutes and subjected to suction filtration under reduced pressure to collect the water-soluble polymer partially neutralized to free acid. The collected water-soluble polymer was wet crystals containing a hydrous alcohol. The wet crystals were transferred to a petri dish, introduced into a fan dryer set at 70°C, and dried for 20 hours to perform cross-linking treatment. The solids obtained after the treatment were pulverized in a mortar and sieved through sieves with openings of 150 pm and 850 µm to collect particles having a particle size of 150 to 850 pm.

(Examples 11 and 12, and Comparative Example 1)

Water-soluble resins were obtained by the same procedure as in Example 10, except that the raw materials and the amounts thereof charged were changed as shown in Table 4.

(Example 14)

A 25 g portion of the water-soluble polymer obtained in Production Example 15 (product wet with hydrous ethanol; wet ratio: 61%) was introduced into a 300 ml beaker, and 80 ml of hydrous ethanol with an ethanol/water ratio = 90/10 (by volume) was further added to disperse the water-soluble polymer. To this dispersion, 12.1 ml of 1N hydrochloric acid was gradually added with a measuring pipette while stirring with a magnetic stirrer. After adding the hydrochloric acid, the mixture was stirred for 15 minutes and subjected to suction filtration under reduced pressure to collect the water-soluble polymer partially neutralized to free acid. The collected water-soluble polymer was wet crystals containing a

hydrous alcohol. The wet crystals were transferred to a petri dish, introduced into a fan dryer set at 150°C, and dried for one hour to perform cross-linking treatment. The solids obtained after the treatment were pulverized in a mortar and sieved through sieves with openings of 150 pm and 850 μm to collect particles having a particle size of 150 to 850 pm.

(Example 16)

A 20 g portion of the water-soluble polymer obtained in Production Example 17 (product wet with hydrous acetone; wet ratio: 61%) was introduced into a 300 ml beaker, and 60 ml of hydrous acetone with an acetone/water ratio = 90/10 (by volume) was further added to disperse the water-soluble polymer. To this dispersion, 19.3 ml of 1N NaOH was gradually added with a measuring pipette while stirring with a magnetic stirrer. After adding the aqueous NaOH solution, the mixture was stirred for 15 minutes and subjected to suction filtration under reduced pressure to collect the water-soluble polymer in which some of the carboxylic acid moieties introduced by maleic anhydride were neutralized to sodium salt. The collected water-soluble polymer was wet crystals containing hydrous acetone. The wet crystals were transferred to a petri dish, introduced into a fan dryer set at 70°C, and dried for 12 hours to perform cross-linking treatment. The solids obtained after the treatment were pulverized in a mortar and sieved through sieves with openings of 150 pm and 850 μm to collect particles having a particle size of 150 to 850 pm.

(4-3) Method including partial neutralization during synthesis of water-soluble polymer

(Example 15)

A 25 g portion (wet crystals) of the water-soluble polymer obtained in Production Example 16 (product wet with hydrous methanol; wet ratio: 63%) was transferred to a petri dish, introduced into a fan dryer set at 70°C, and dried for 12 hours to perform cross-linking treatment. The solids obtained after the treatment were pulverized in a mortar and sieved through sieves with openings of 150 pm and 850 μm to collect particles having a particle size of 150 to 850 μm.

(5) Method for evaluating water-soluble polymer

(5-1) Total acid value of water-soluble polymer

About 0.3 g of the water-soluble polymer was precisely weighed into a 100 ml beaker and dissolved in 40 ml of ion-exchanged water. This aqueous solution was set in a potentiometric titrator (AT-610 available from Kyoto Electronics Manufacturing Co., Ltd.) equipped with glass electrodes (C-171 available from Kyoto Electronics Manufacturing Co., Ltd.). A sample in which all groups formed a sodium salt would exhibit a potential of approximately 30 mV or less at this stage. Therefore, 1N hydrochloric acid was added until the potential reached 320 mV or more to convert all carboxylic acid groups in the water-soluble polymer into the free acid form (resulting in an excess of hydrochloric acid). After confirming that the potential was 320 mV or more, neutralization titration was performed with a 0.1N aqueous NaOH solution. In this titration, two inflection points were detected, with the first inflection point being near 220 mV and the second inflection point being near 0 to -30 mV. The former is the neutralization point of the excess hydrochloric acid in the sample and the latter is the neutralization point of the carboxylic acid in the water-soluble polymer. Thus, the total acid value is calculated by the following equation 1:

$$\text{Total acid value (mg KOH/g)} = [\{(Vb - Va) \times 0.1 \times fa \times 56.11\} \div Sa]/(1 - wr) \quad \text{(Equation 1)}$$

wherein

Va represents the volume (ml) of 0.1N NaOH consumed up to the first inflection point;
Vb represents the volume (ml) of 0.1N NaOH consumed up to the second inflection point;
fa represents the titer of 0.1N NaOH;
Sa represents the amount of the sample collected; and
wr represents the wet ratio of the water-soluble polymer measured by the method described later.

(5-2) Free acid value of water-soluble polymer when acid treatment or alkali treatment is successively followed by production of cross-linked polymer (Examples 10 to 12, Example 14, Example 16, and Comparative Example 1)

Since acid treatment or alkali treatment is successively followed by the production of a cross-linked polymer, it is difficult to perform direct quantification by titration. Therefore, the value calculated as follows is defined as the free acid value.

When acid treatment is performed with an acid having a pKa lower than the carboxylic acid of the water-soluble polymer, the amount of acid added during the acid treatment is substantially equal to the free acid value. Therefore, the free acid value is calculated by the following equation 2:

$$\text{Free acid value (mg KOH/g)} = (Vc \times N \times fb \times 56.11) \div (Sb - Vc \times N \times fb \times 22) \quad \text{(Equation 2)}$$

wherein

Vc represents the volume (ml) of the aqueous acid solution used in the acid treatment;
N represents the normality of the aqueous acid solution;
fb represents the titer of the aqueous acid solution; and
Sb represents the (net) weight of the water-soluble polymer charged in the acid treatment.

When alkali treatment with sodium hydroxide is performed, the free acid will be neutralized by the alkali. Therefore, the free acid value is calculated by the following equation 3:

$$\text{Free acid value (mg KOH/g)} = \{(Tav \div 56.11) \times Sc - Vd \times N \times fc) \div (Sc + Vd \times N \times fc \times 22)\} \times 56.11 \quad \text{(Equation 3)}$$

wherein

Tav represents the total acid value (mg KOH/g) of the water-soluble polymer;
Vd represents the volume (ml) of the aqueous sodium hydroxide solution used in the alkali treatment;
N represents the normality of the aqueous sodium hydroxide solution;
fc represents the titer of the aqueous sodium hydroxide solution; and
Sc represents the (net) weight of the water-soluble polymer charged in the alkali treatment.

(5-3) Free acid value of water-soluble polymer when partial neutralization is performed during synthesis of water-soluble polymer (Example 15)

Neutralization titration is performed by the method described above in "(5-1) Total acid value of water-soluble polymer" without adding 1N hydrochloric acid before the titration. In this case, one inflection point is detected, and the free acid value is calculated by the following equation 4:

$$\text{Free acid value (mg KOH/g)} = [Va \times 0.1 \times fa \times 56.11 \div Sa]/(1 - wr) \quad \text{(Equation 4)}$$

wherein

Va represents the volume (ml) of 0.1N NaOH consumed up to the inflection point;
fa represents the titer of 0.1N NaOH;
Sa represents the amount of the sample collected; and
wr represents the wet ratio of the water-soluble polymer.

(5-4) Wet ratio of water-soluble polymer

The term "wet ratio" refers to the rate (%) of reduction in weight relative to the initial weight of the sample when the sample is dried at a drying temperature of 130°C and measured with a halogen moisture meter. In the examples, 0.5 to 1.0 g of the water-soluble polymer was set in a halogen moisture meter HC103 available from Mettler Toledo and measured at a drying temperature of 130°C and a switch-off criterion of 1 mg/50 seconds in % MC mode (a mode that displays MC value = (initial weight of sample - dry weight of sample) ÷ initial weight of sample × 100). The displayed MC value was taken as the wet ratio.

(5-5) Degree of etherification of water-absorbing polymer

$$\text{Degree of etherification} = (162 \times TAV) \div (56100 - 80 \times TAV)$$

In the equation, TAV represents the total acid value (unit: mg KOH/g) of the water-soluble polymer.

(5-6) Biomass content of water-soluble polymer

The term "biomass content" refers to the percentage (% by mass) of elements derived from natural resources among the elements constituting the water-soluble polymer. The biomass content was determined by the following formula using the degree of etherification. It should be noted that the water-absorbing resin obtained by partially neutralizing the water-soluble polymer followed by drying can have a slightly higher biomass content because the sodium salt is removed from the acidic group by the partial neutralization.

((Molecular weight of glucose unit) - Degree of etherification × 1)/((Molecular weight of glucose unit) + (Degree of etherification × (Molecular weight of acidic group - 1))) × 100

In the numerator of the formula, the molecular weight of the starch monomer (glucose unit) is 162, from which a value of "Degree of etherification × 1" is subtracted because a hydrogen atom is removed upon the introduction of the acidic group.

In the denominator of the formula, the molecular weight of the acidic group which is a carboxymethyl group (sodium salt) is 81, while the molecular weight of the acidic group which is a carboxyhexyl group is 137. A value obtained by subtracting one from the molecular weight is used because a hydrogen atom is removed upon the introduction of the acidic group.

(6) Method for evaluating water-absorbing resin (6-1) Water absorption rate under no pressure (for physiological saline)

A 1.0 g portion of each measurement sample was put in a tea bag (20 cm long × 10 cm wide) made of a nylon mesh having an opening of 63 pm (JIS Z 8801-1:2006) and immersed in 1,000 ml of physiological saline (salt concentration: 0.9% by weight) for three hours without stirring. Thereafter, the tea bag was hung for 10 minutes for draining. The weight (h1) including that of the tea bag was measured, and the water retention amount was determined by the following equation. Here, the temperatures of the physiological saline used and the measurement atmosphere were each 25°C ± 2°C.

$$FSC\ (g/g) = (h1) - (h2)$$

Here, (h2) represents the weight of the tea bag containing no measurement sample measured by the same procedure as above, and FSC is an abbreviation for Free Swell Capacity, which means the free swell rate and indicates the water absorption rate under no pressure.

(6-2) Water absorption rate under no pressure (for ion-exchanged water)

The weight (h1') including that of the tea bag after immersion was measured as described for the water absorption rate under no pressure (for physiological saline), except that 0.2 g of the measurement sample was put in the tea bag and ion-exchanged water was used instead of physiological saline. Then, the water retention amount was determined by the following equation. Here, (h2') represents the weight of the tea bag containing no measurement sample measured by the same procedure as above.

$$FSC\ (g/g) = \{(h1') - (h2')\}/0.2$$

(6-3) Water retention rate (for physiological saline)

After measuring the water absorption rate under no pressure described above, the whole tea bag was placed in a centrifuge and dehydrated by centrifugation at 150 G for 90 seconds to remove an excess liquid component. The weight (h3) including that of the tea bag was measured, and the water retention amount was determined by the following equation.

$$CRC\ (g/g) = (h3) - (h4)$$

Here, (h4) represents the weight of the tea bag containing no measurement sample measured by the same

procedure as above, and CRC is an abbreviation for Centrifuge Retention Capacity, which means the centrifuge retention capacity and indicates the water retention rate.

(6-4) Water retention rate (for ion-exchanged water)

After measuring the water absorption rate under no pressure described above, the whole tea bag was placed in a centrifuge and dehydrated by centrifugation at 150 G for 90 seconds to remove an excess liquid component. The weight (h3') including that of the tea bag was measured, and the water retention amount was determined by the following equation.

$$CRC\ (g/g) = \{(h3') - (h4')\}/0.2$$

Here, (h4') represents the weight of the tea bag containing no measurement sample measured by the same procedure as above.

(6-5) Gel properties of water-absorbing resin

After the water retention rate test for physiological saline described in section (6-3), the gel was taken out of the tea bag, and the appearance of the gel was visually evaluated on the following six-point scale. FIG. 1 shows photographs of gel conditions.

1: Soluble in water
2: Semi-soluble in water
3: Insoluble in water and watery
4: Insoluble in water and a little watery
5: Insoluble in water and soft
6: Insoluble in water and firm

(6-6) Alkali degradability

A 0.5 g portion of the water-absorbing resin and 9.5 g of a 1% aqueous NaOH solution were weighed into a 13.5 ml screw tube bottle, and they were dissolved by stirring with a mix rotor for 60 minutes. Next, an about 50 mm × 50 mm square 30-mesh sieve (opening: 500 pm) was provided and the weight (Wm1) of the sieve was measured. The mixture was subjected to natural filtration through the weighed sieve. After the filtration, the top of the mesh was washed with about 5 ml of ion-exchanged water. After the washing, the sieve was dried with a fan dryer at 120°C for two hours. Then, the weight (Wm2) of the dried sieve was measured, and the 30-mesh pass residue was calculated by the following equation. If the water-absorbing resin has been completely dissolved, the 30-mesh pass residue is 0%. If the water-absorbing resin has not been dissolved and the gel remains, the 30-mesh pass exceeds 0%.

$$30\text{-Mesh pass residue } (\%) = (Wm2 - Wm1)/0.5 \times 100$$

(7) Evaluation of water-absorbing resin

The water absorption performance and gel properties of the water-absorbing resins obtained in Examples 1 to 16 and Comparative Examples 1 to 3 were evaluated. Table 4 shows the results.

[Table 4]

| | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Ex. 14 | Ex. 15 | Ex. 16 | Comp. Ex. 1 | Comp. Ex. 2 | Comp. Ex. 3 |
|---|---|---|---|---|---|---|---|---|---|---|---|---|---|---|---|---|---|---|---|---|
| Partially hydrolyzed starch | | Prod. Ex. 1 | Prod. Ex. 1 | Prod. Ex. 1 | Prod. Ex. 1 | Prod. Ex. 1 | Prod. Ex. 1 | Prod. Ex. 1 | Prod. Ex. 1 | Prod. Ex. 1 | Prod. Ex. 3 | Prod. Ex. 4 | Prod. Ex. 2 | Prod. Ex. 5 | Prod. Ex. 4 | Prod. Ex. 7 | Prod. Ex. 6 | Prod. Ex. 2 | Prod. Ex. 1 | Prod. Ex. 1 |
| Water-soluble polymer | | Prod. Ex. 9 | Prod. Ex. 9 | Prod. Ex. 10 | Prod. Ex. 8 | Prod. Ex. 8 | Prod. Ex. 8 | Prod. Ex. 9 | Prod. Ex. 9 | Prod. Ex.10 | Prod. Ex. 12 | Prod. Ex. 13 | Prod. Ex. 11 | Prod. Ex. 14 | Prod. Ex. 15 | Prod. Ex. 16 | Prod. Ex. 17 | Prod. Ex. 11 | Prod. Ex. 8 | Prod. Ex. 8 |
| Water-soluble polymer (g) | | 25 | 25 | 25 | 25 | 25 | 25 | 25 | 25 | 25 | 25 | 25 | 25 | 25 | 25 | 25 | 20 | 25 | 25 | 25 |
| Wet ratio (%) of water-soluble polymer | | 67 | 67 | 67 | 67 | 67 | 67 | 67 | 67 | 67 | 71 | 71 | 71 | 74 | 61 | 63 | 61 | 71 | 67 | 67 |
| Hydrous methanol (ml) | | 0 | 0 | 0 | 0 | 0 | 0 | 0 | 0 | 0 | 100 | 100 | 100 | 0 | 80*2 | 0 | 0 | 100 | 0 | 0 |
| Hydrous acetone (ml) | | 0 | 0 | 0 | 0 | 0 | 0 | 0 | 0 | 0 | 0 | 0 | 0 | 0 | 0 | 0 | 60 | 0 | 0 | 0 |
| 1N Hydrochloric acid (ml) | | 0 | 0 | 0 | 0 | 0 | 0 | 0 | 0 | 0 | 1.1 | 1.1 | 1.5 | 0 | 12.1 | 0 | 0 | 1.5 | 0 | 0 |
| 1N NaOH (ml) | | 0 | 0 | 0 | 0 | 0 | 0 | 0 | 0 | 0 | 0 | 0 | 0 | 0 | 0 | 0 | 10.3 | 0 | 0 | 0 |
| Drying temperature (℃) | | 100 | 130 | 130 | 130 | 150 | 150 | 150 | 150 | 150 | 70 | 70 | 100 | 150 | 150 | 70 | 70 | 50 | 70 | 90 |
| Duration of drying (hour) | | 20 | 3 | 20 | 20 | 20 | 3 | 3 | 20 | 3 | 20 | 20 | 3 | 3 | 1 | 12 | 12 | 20 | 6 | 3 |
| Solid content (%) | | 97.1 | 97.0 | 98.3 | 97.8 | 97.5 | 97.5 | 97.0 | 97.6 | 97.7 | 91.3 | 93.4 | 97.5 | 97.7 | 96.3 | 92.9 | 97.5 | 82.7 | 83.1 | 74.8 |
| Free acid value (mg KOH/g) | | 0.0 | 0.0 | 0.0 | 0.0 | 0.0 | 0.0 | 0.0 | 0.0 | 0.0 | 8.0 | 8.0 | 11.0 | 0.0 | 11.0 | 35.0 | 16.7 | 11.0 | 0.0 | 0.0 |
| Water absorption performance | Ion-exchanged water FSC | 322 | 212 | 153 | 190 | 114 | 215 | 138 | 70 | 159 | 199 | 192 | 148 | 151 | 62 | 160 | 40 | 0 | 0 | 0 |
| | Ion-exchanged water CRC | 102 | 181 | 112 | 162 | 79 | 185 | 101 | 49 | 122 | 153 | 150 | 123 | 113 | 39 | 63 | 21 | 0 | 0 | 0 |
| | Physiological saline FSC | 39 | 45 | 37 | 44 | 35 | 47 | 37 | 29 | 40 | 45 | 41 | 33 | 34 | 20 | 25 | 11 | 0 | 0 | 0 |
| | Physiological saline CRC | 35 | 41 | 35 | 42 | 30 | 44 | 33 | 21 | 37 | 41 | 38 | 30 | 30 | 14 | 22 | 7 | 0 | 0 | 0 |
| Gel properties | | 3 | 5 | 6 | 5 | 6 | 5 | 6 | 6 | 6 | 4 | 5 | 4 | 6 | 6 | 3 | 6 | 1 | 1 | 1 |
| Alkali degradability (30-mesh pass residue (%)) | | 0 | 0 | 0 | 0 | 0 | 0 | 0 | 0 | 0 | 0 | 0 | 0 | 0 | 0 | 0 | 0 | *1 | *1 | *1 |

Ex.: Example
Comp. Ex.: Comparative Example
Prod. Ex.: Production Example

*1: Comparative examples were soluble in water and thus not evaluated.
*2: Hydrous ethanol was used instead of hydrous methanol.

In Comparative Examples 1 to 3, the water-soluble polymers in a dry state had a solid content of less than 90%. Thus, they were not gelled and did not exhibit water absorption performance. In Examples 1 to 16, the solid contents of the water-soluble polymers in a dry state were adjusted to be 90% or more. Thus, water-insoluble water-absorbing resins

could be obtained, and they achieved sufficient water absorption performance.

(8) Production of partially degraded modified starch (Production Examples 18 to 22)

Partially degraded modified starches were produced as described below. Table 5 shows the weight average molecular weight and dispersity of each partially degraded product.

(Production Example 18) Partially degraded starch derived from tapioca starch acetate

Tapioca starch acetate was suspended in city water to a concentration of 45% (w/w), and then 1N sodium hydroxide was added thereto to adjust the pH to 6.0 to obtain a starch milk. To the starch milk were added 3 units of the crude enzyme solution of amylomaltase per gram of the starch solids. The mixture was stirred at room temperature for 30 minutes and then reacted at 90°C for five hours while stirring.

(Production Example 19) Partially degraded starch derived from acetylated oxidized tapioca starch

Acetylated oxidized tapioca starch was suspended in city water to a concentration of 45% (w/w), and then 1N sodium hydroxide was added thereto to adjust the pH to 6.0 to obtain a starch milk. To the starch milk were added 3 units of the crude enzyme solution of amylomaltase per gram of the starch solids. The mixture was stirred at room temperature for 30 minutes and then reacted at 90°C for five hours while stirring.

(Production Example 20) Partially degraded starch derived from acetylated tapioca starch

Acetylated oxidized tapioca starch was suspended in city water to a concentration of 45% (w/w), and then 1N sodium hydroxide was added thereto to adjust the pH to 6.0 to obtain a starch milk. To the starch milk were added 5 units of the crude enzyme solution of amylomaltase per gram of the starch solids. The mixture was stirred at room temperature for 30 minutes and then reacted at 90°C for five hours while stirring.

(Production Example 21) Partially degraded starch derived from hydroxypropyl starch

Hydroxypropyl starch was suspended in city water to a concentration of 45% (w/w), and then 1N sodium hydroxide was added thereto to adjust the pH to 6.0 to obtain a starch milk. To the starch milk were added 3 units of the crude enzyme solution of amylomaltase per gram of the starch solids. The mixture was stirred at room temperature for 30 minutes and then reacted at 90°C for five hours while stirring.

(Production Example 22) Partially degraded starch derived from hydroxypropyl distarch phosphate

Hydroxypropyl distarch phosphate was suspended in city water to a concentration of 30% (w/w), and then 1N sodium hydroxide was added thereto to adjust the pH to 6.0 to obtain a starch milk. To the starch milk were added 3 units of the crude enzyme solution of amylomaltase per gram of the starch solids. The mixture was stirred at room temperature for 30 minutes and then reacted at 90°C for five hours while stirring.

[Table 5]

| | Production Example 18 | Production Example 19 | Production Example 20 | Production Example 21 | Production Example 22 |
|---|---|---|---|---|---|
| Mw ($\times 10^4$) | 465 | 62 | 456 | 256 | 204 |
| Dispersity | 19.1 | 7.4 | 8.9 | 8.1 | 8.4 |

(9) Production of water-soluble polymer

(Production Example 23)

A 100 g portion of a 45% by weight aqueous solution of the partially hydrolyzed starch produced in Production Example 18 (0.83 mol of hydroxyl groups of the partially degraded starch) was charged into a 500 ml separable flask equipped with a stirrer, a thermometer, and a condenser tube. Next, 34.4 g of a 48.8% aqueous sodium hydroxide solution

(0.42 mol, 0.5 equivalents relative to the hydroxyl groups of the partially degraded starch) was charged into the flask, and stirred at 60°C or lower until the solution became completely homogeneous. After confirming that the solution was homogeneous, an aqueous solution prepared by dissolving 18.9 g of monochloroacetic acid (0.2 mol, 0.2 equivalents relative to the hydroxyl groups of the partially degraded starch) in 25 g of ion-exchanged water was charged dropwise at 50°C to 60°C over 30 minutes. After charging the aqueous monochloroacetic acid solution, the temperature was adjusted to 45°C to 50°C, and the mixture was stirred for 10 hours. The end point of the reaction was determined by sampling the reaction liquid and measuring the chlorine ion content in the reaction liquid by potentiometric titration with a 0.01N silver nitrate solution, and was defined as the point at which the chlorine ion content reached at least 98% of the calculated chlorine ion content of 4.0%, which corresponds to the chlorine ion content when all the monochloroacetic acid has reacted. In this production example, the chlorine content was found to be 4.0%.

After completion of the reaction, the reaction liquid was diluted with 400 g of ion-exchanged water. The diluted reaction liquid was cooled to room temperature and added to 5 L of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 1 L of hydrous methanol with a methanol/water ratio of 80/20 (by volume) and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01N silver nitrate solution, and the washing process was repeated until the chlorine content was less than 1%. The resulting water-soluble polymer had a total acid value of 146 mg KOH/g and a degree of etherification calculated from the total acid value of 0.53. The water-soluble polymer also had a weight average molecular weight of $18.6 \times 10^6$ and a dispersity of 19.7.

(Production Examples 24 to 27)

Water-soluble polymers were produced by the same procedure as in Production Example 23, except that the raw materials and the amounts thereof charged and the reaction conditions were changed as shown in Table 6. Table 6 shows the total acid value, degree of etherification, weight average molecular weight, and dispersity of each water-soluble polymer.

[Table 6]

| Water-soluble polymer | | Production Example 23 | Production Example 24 | Production Example 25 | Production Example 26 | Production Example 27 |
|---|---|---|---|---|---|---|
| Partially hydrolyzed starch 1 | | Production Example 18 | Production Example 19 | Production Example 20 | Production Example 21 | Production Example 22 |
| | Concentration of aqueous solution (%) | 45 | 45 | 45 | 45 | 30 |
| | Amount of aqueous solution (g) | 100.0 | 100.0 | 100.0 | 100.0 | 100.0 |
| Alkaline agent | 48.8% NaOH aqueous solution (g) | 34.4 | 34.4 | 34.4 | 34.4 | 45.5 |
| Aqueous solution containing carboxyl alkyl group-containing compound | Monochloroacetic acid (g) | 18.9 | 18.9 | 18.9 | 18.9 | - |
| | Sodium monochloroacetate | - | - | - | - | 64.7 |
| | Water for dissolving monochloroacetic acid or sodium monochloroacetate (g) | 25.0 | 20.0 | 25.0 | 20.0 | 83.7 |
| Reaction temperature (°C) | | 45 to 50 | 45 to 50 | 45 to 50 | 45 to 50 | 80 to 90 |
| Water for diluting reaction liquid (g) | | 400 | 300 | 400 | 500 | 500 |
| Methanol for reprecipitation (L) | | 5.0 | 3.0 | 4.0 | 4.0 | 4.0 |
| Hydrous methanol for washing (L) | | 1.0 | 1.0 | 1.0 | 1.0 | 1.0 |

(continued)

| Water-soluble polymer | | Production Example 23 | Production Example 24 | Production Example 25 | Production Example 26 | Production Example 27 |
|---|---|---|---|---|---|---|
| Physical properties of water-soluble polymer | Mw ($\times 10^6$) | 18.6 | 1.0 | 16.7 | 6.7 | 5.4 |
| | Mw/Mn | 197 | 11.5 | 30.0 | 13.0 | 10.2 |
| | Total acid value (mg KOH/g) | 146 | 155 | 144 | 132 | 189 |
| | Degree of etherification | 0.53 | 0.58 | 0.52 | 0.47 | 0.75 |

(10) Production of water-absorbing resin

(Example 17)

A 25 g portion of the water-soluble polymer obtained in Production Example 23 (product wet with hydrous methanol; wet ratio: 65%) was introduced into a 300 ml beaker, and 120 ml of hydrous methanol with a methanol/water ratio = 80/20 (by volume) was further added to disperse the water-soluble polymer. To this dispersion, 0.8 ml of 1N hydrochloric acid was gradually added with a measuring pipette while stirring with a magnetic stirrer. After adding the hydrochloric acid, the mixture was stirred for 15 minutes and subjected to suction filtration under reduced pressure to collect the water-soluble polymer partially neutralized to free acid. The collected water-soluble polymer was wet crystals containing a hydrous alcohol. The wet crystals were transferred to a petri dish, introduced into a fan dryer set at 70°C, and dried for 12 hours to perform cross-linking treatment. The solids obtained after the treatment were pulverized in a mortar and sieved through sieves with openings of 150 pm and 850 μm to collect particles having a particle size of 150 to 850 pm.

(Examples 18 to 25)

Water-absorbing resins were obtained by the same procedure as in Example 17, except that the raw materials and the amounts thereof charged and the reaction conditions were changed as shown in Table 7. Table 7 shows the free acid value, water absorption performance, gel properties, and alkali degradability of each water-absorbing resin.

[Table 7]

| | | Example 17 | Example 18 | Example 19 | Example 20 | Example 21 | Example 22 | Example 23 | Example 24 | Example 25 |
|---|---|---|---|---|---|---|---|---|---|---|
| Partially hydrolyzed starch | | Production Example 18 | Production Example 18 | Production Example 19 | Production Example 20 | Production Example 20 | Production Example 21 | Production Example 21 | Production Example 22 | Production Example 22 |
| Water-soluble polymer | | Production Example 23 | Production Example 23 | Production Example 24 | Production Example 25 | Production Example 25 | Production Example 26 | Production Example 26 | Production Example 27 | Production Example 27 |
| Water-soluble polymer (g) | | 25 | 25 | 25 | 25 | 25 | 25 | 25 | 25 | 25 |
| Wet ratio (%) of water-soluble polymer | | 65 | 65 | 69 | 66 | 66 | 62 | 62 | 55 | 55 |
| Hydrous methanol (ml) | | 120 | 120 | 100 | 120 | 80 | 100 | 80 | 100 | 120 |
| 1N Hydrochloric acid (ml) | | 0.8 | 1.4 | 7.8 | 1.7 | 0 | 1.9 | 0 | 2.2 | 3.3 |
| Acetic acid (ml) | | 0 | 0 | 0 | 0 | 0.06 | 0 | 0.11 | 0 | 0 |
| Drying temperature (°C) | | 70 | 80 | 70 | 70 | 90 | 70 | 100 | 70 | 70 |
| Duration of drying (hour) | | 12 | 10 | 19 | 12 | 20 | 12 | 6 | 12 | 12 |
| Solid content (%) | | 93.1 | 95.1 | 95.8 | 94.3 | 92.1 | 96.3 | 95.4 | 94.6 | 95.2 |
| Free acid value (mg KOH/g) | | 6.0 | 11.0 | 56.0 | 11.0 | 7.0 | 11.0 | 11.0 | 11.0 | 16.0 |
| Water absorption performance | Ion-exchanged water FSC | 400 | 292 | 83 | 281 | 374 | 277 | 232 | 266 | 249 |
| | Ion-exchanged water CRC | 197 | 238 | 57 | 230 | 236 | 206 | 191 | 204 | 174 |
| | Physiological saline FSC | 48 | 42 | 22 | 47 | 53 | 46 | 41 | 54 | 48 |
| | Physiological saline CRC | 45 | 39 | 18 | 44 | 49 | 43 | 40 | 49 | 43 |
| Gel properties | | 3 | 5 | 6 | 5 | 4 | 3 | 3 | 3 | 3 |
| Alkali degradability (30-mesh pass residue (%)) | | 0 | 0 | 0 | 0 | 0 | 0 | 0 | 0 | 0 |

Also, in Examples 17 to 25 using a modified starch as a raw material, the solid contents of the water-soluble polymers in a dry state were adjusted to 90% or more. Thus, water-insoluble water-absorbing resins could be obtained, and they achieved sufficient water absorption performance.

(Production Example 28) Partially degraded starch derived from tapioca starch (1)

Tapioca starch was suspended in city water to a concentration of 45% (w/w), and then 1N sodium hydroxide was added thereto to adjust the pH to 6.0 to obtain a starch milk. To the starch milk were added 8.0 units of the crude enzyme solution of amylomaltase per gram of the starch solids. The mixture was stirred at room temperature for 30 minutes and then reacted at 95°C for six hours while stirring to prepare a liquefied starch. The obtained partially degraded starch had a weight average molecular weight of 2,610,000 and a dispersity of 7.9.

(Production Example 29) Production (1) of water-soluble polymer derived from tapioca starch

A 100 g portion of a 45% by weight aqueous solution of the partially degraded starch derived from tapioca starch obtained in Production Example 28 (0.83 mol of hydroxyl groups of the partially degraded starch) was charged into a 500 ml separable flask equipped with a stirrer, a thermometer, and a condenser tube. Next, 34.0 g of a 48.8% aqueous sodium hydroxide solution (0.42 mol, 0.50 molar equivalents per mole of hydroxyl groups of the partially degraded starch) was charged into the flask, and stirred at 60°C or lower until the solution became completely homogeneous. After confirming that the solution was homogeneous, an aqueous solution prepared by dissolving 19.8 g of monochloroacetic acid (0.21 mol, 0.3 molar equivalents per mole of hydroxyl groups of the partially degraded starch) in 50 g of ion-exchanged water was charged dropwise at 50°C to 60°C over 30 minutes. After charging the aqueous monochloroacetic acid solution, the temperature was adjusted to 45°C to 50°C, and the mixture was stirred for 12 hours. The end point of the reaction was determined by sampling the reaction liquid and measuring the chlorine ion content in the reaction liquid by potentiometric titration with a 0.01N aqueous silver nitrate solution, and was defined as the point at which the chlorine ion content reached at least 98% of the calculated chlorine ion content of 3.7%, which corresponds to the chlorine ion content when all the monochloroacetic acid has reacted. In this production example, the chlorine content was found to be 3.7%.

After completion of the reaction, the reaction liquid was diluted with 900 g of ion-exchanged water. The diluted reaction liquid was cooled to room temperature and added to 6000 ml of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 1000 ml of hydrous methanol with a methanol/water ratio of 80/20 (by volume) and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01N aqueous silver nitrate solution, and the washing process was repeated until the chlorine content was less than 1%. The resulting water-soluble polymer had a total acid value of 130 mg KOH/g, a degree of etherification calculated from the total acid value of 0.46, a weight average molecular weight of 8,120,000, a dispersity of 13.7, and a biomass content of 81%.

(Production Example 30) Production (2) of water-soluble polymer derived from tapioca starch

A 95 g portion of a 45% by weight aqueous solution of the partially degraded starch derived from tapioca starch obtained in Production Example 28 (0.79 mol of hydroxyl groups of the partially degraded starch) and 2.5 g of tapioca starch (net tapioca starch content: 2.2 g, 0.04 mol of hydroxyl groups of the tapioca starch) were charged into a 500 ml separable flask equipped with a stirrer, a thermometer, and a condenser tube. Next, 34.0 g of a 48.8% aqueous sodium hydroxide solution (0.42 mol, 0.50 molar equivalents per mole of hydroxyl groups of the partially degraded starch) was charged into the flask, and stirred at 60°C or lower until the solution became completely homogeneous. After confirming that the solution was homogeneous, an aqueous solution prepared by dissolving 19.8 g of monochloroacetic acid (0.21 mol, 0.3 molar equivalents per mole of hydroxyl groups of the partially degraded starch) in 50 g of ion-exchanged water was charged dropwise at 50°C to 60°C over 30 minutes. After charging the aqueous monochloroacetic acid solution, the temperature was adjusted to 45°C to 50°C, and the mixture was stirred for 12 hours. The end point of the reaction was determined by sampling the reaction liquid and measuring the chlorine ion content in the reaction liquid by potentiometric titration with a 0.01N aqueous silver nitrate solution, and was defined as the point at which the chlorine ion content reached at least 98% of the calculated chlorine ion content of 3.7%, which corresponds to the chlorine ion content when all the monochloroacetic acid has reacted. In this production example, the chlorine content was found to be 3.7%.

After completion of the reaction, the reaction liquid was diluted with 900 g of ion-exchanged water. The diluted reaction liquid was cooled to room temperature and added to 6000 ml of methanol over about 30 minutes to reprecipitate a

water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 1000 ml of hydrous methanol with a methanol/water ratio of 80/20 (by volume) and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01N aqueous silver nitrate solution, and the washing process was repeated until the chlorine content was less than 1%. The resulting water-soluble polymer had a total acid value of 132 mg KOH/g, a degree of etherification calculated from the total acid value of 0.47, a weight average molecular weight of 8,050,000, a dispersity of 14.7, and a biomass content of 81%.

(Production Example 31) Partially degraded starch derived from tapioca starch (2)

Tapioca starch was suspended in city water to a concentration of 45% (w/w), and then 1N sodium hydroxide was added thereto to adjust the pH to 6.0 to obtain a starch milk. To the starch milk were added 4.0 units of the crude enzyme solution of amylomaltase per gram of the starch solids. The mixture was stirred at room temperature for 30 minutes and then reacted at 85°C for five hours while stirring to prepare a liquefied starch. The obtained partially degraded starch had a weight average molecular weight of 4,010,000 and a dispersity of 14.1.

(Production Example 32) Production (3) of water-soluble polymer derived from tapioca starch

A 100 g portion of a 45% by weight aqueous solution of the partially degraded starch derived from tapioca starch obtained in Production Example 31 (0.83 mol of hydroxyl groups of the partially degraded starch) was charged into a 500 ml separable flask equipped with a stirrer, a thermometer, and a condenser tube. Next, 59.5 g of a 48.8% aqueous sodium hydroxide solution (0.73 mol, 0.87 molar equivalents per mole of hydroxyl groups of the partially degraded starch) was charged into the flask, and stirred at 60°C or lower until the solution became completely homogeneous. After confirming that the solution was homogeneous, an aqueous solution prepared by dissolving 32.7 g of monochloroacetic acid (0.35 mol, 0.4 molar equivalents per mole of hydroxyl groups of the partially degraded starch) in 80 g of ion-exchanged water was charged dropwise at 50°C to 60°C over 30 minutes. After charging the aqueous monochloroacetic acid solution, the temperature was adjusted to 45°C to 50°C, and the mixture was stirred for 12 hours. The end point of the reaction was determined by sampling the reaction liquid and measuring the chlorine ion content in the reaction liquid by potentiometric titration with a 0.01N aqueous silver nitrate solution, and was defined as the point at which the chlorine ion content reached at least 98% of the calculated chlorine ion content of 4.6%, which corresponds to the chlorine ion content when all the monochloroacetic acid has reacted. In this production example, the chlorine content was found to be 4.6%.

After completion of the reaction, the reaction liquid was diluted with 1000 g of ion-exchanged water. The diluted reaction liquid was cooled to room temperature and added to 7000 ml of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 1000 ml of hydrous methanol with a methanol/water ratio of 80/20 (by volume) and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01N aqueous silver nitrate solution, and the washing process was repeated until the chlorine content was less than 1%. The resulting water-soluble polymer had a total acid value of 179 mg KOH/g, a degree of etherification calculated from the total acid value of 0.69, a weight average molecular weight of 9,600,000, a dispersity of 36.7, and a biomass content of 74% .

(Example 26) Production of liquid-absorbing resin

A 25 g portion of the water-soluble polymer obtained in Production Example 29 (product wet with hydrous methanol; wet ratio: 65%) was introduced into a 500 ml beaker, and 200 ml of hydrous methanol with a methanol/water ratio = 80/20 (by volume) was further added to disperse the water-soluble polymer. To this dispersion, 1.7 ml of a 1N aqueous hydrochloric acid solution was gradually added with a measuring pipette while stirring with a magnetic stirrer. After adding the hydrochloric acid, the mixture was stirred for one hour and subjected to suction filtration under reduced pressure to collect the water-soluble polymer partially neutralized to free acid. The collected water-soluble polymer was wet crystals containing a hydrous alcohol. The wet crystals were transferred to a petri dish, introduced into a fan dryer set at 70°C, and dried for 20 hours to perform cross-linking treatment. The solids obtained after the treatment were pulverized in a mortar and sieved through sieves with openings of 150 pm and 850 μm to collect liquid-absorbing resin particles having a particle

size of 150 to 850 pm.

(Example 27) Production of liquid-absorbing resin

Liquid-absorbing resin particles were collected by the same procedure as in Example 26, except that the 1N aqueous hydrochloric acid solution was changed to 0.23 g of DL-malic acid.

(Example 28) Production of liquid-absorbing resin

Liquid-absorbing resin particles were collected by the same procedure as in Example 26, except that the water-soluble polymer was changed to 25 g of the water-soluble polymer obtained in Production Example 30 (product wet with hydrous methanol; wet ratio: 660).

(Example 29) Production of liquid-absorbing resin

Liquid-absorbing resin particles were collected by the same procedure as in Example 26, except that the water-soluble polymer was changed to 20 g of the water-soluble polymer obtained in Production Example 32 (product wet with hydrous methanol; wet ratio: 69%), the amount of the 1N aqueous hydrochloric acid solution was changed to 0.7 ml, and the cross-linking treatment was performed by introducing the wet crystals into a fan dryer set at 85°C and drying them for 10 hours.

[Table 8]

| | | Example 26 | Example 27 | Example 28 | Example 29 |
|---|---|---|---|---|---|
| Partially hydrolyzed starch | | Production Example 28 | Production Example 28 | Production Example 28 | Production Example 31 |
| Water-soluble polymer | | Production Example 29 | Production Example 29 | Production Example 30 | Production Example 32 |
| Water-soluble polymer (g) | | 25 | 25 | 25 | 20 |
| Wet ratio (%) of water-soluble polymer | | 65 | 65 | 66 | 69 |
| Hydrous methanol (ml) | | 200 | 200 | 200 | 200 |
| 1N Hydrochloric acid (ml) | | 1.7 | 0 | 1.7 | 0.7 |
| DL-malic acid (g) | | 0 | 0.23 | 0 | 0 |
| Drying temperature (°C) | | 70 | 70 | 70 | 85 |
| Duration of drying (hour) | | 20 | 20 | 20 | 10 |
| Solid content (%) | | 95.9 | 94.8 | 94.5 | 95.4 |
| Free acid value (mg KOH/g) | | 11.0 | 22.0 | 11.0 | 6.3 |
| Water absorption performance | Ion-exchanged water FSC | 290 | 352 | 458 | 405 |
| | Ion-exchanged water CRC | 224 | 201 | 100 | 147 |
| | Physiological saline FSC | 48 | 45 | 58 | 69 |
| | Physiological saline CRC | 45 | 41 | 45 | 60 |
| Gel properties | | 5 | 4 | 3 | 3 |
| Alkali degradability (30-mesh pass residue (%)) | | 0 | 0 | 0 | 0 |

Also, in Examples 26, 27, and 29 using tapioca starch as a raw material, the solid contents of the water-soluble

polymers in a dry state were adjusted to 90% or more. Thus, water-insoluble water-absorbing resins could be obtained, and they achieved sufficient water absorption performance. In Example 28, the water-soluble polymer was prepared (Production Example 30) by mixing a partially degraded starch derived from tapioca starch (Production Example 28) with undegraded tapioca starch, and then introducing an acidic group. The resulting water-absorbing resin shows a high water absorption rate under no pressure (FSC) for ion-exchanged water.

## Claims

1. A method for producing a water-absorbing resin that forms a physical gel upon absorption of water, the method comprising:

   (a1) reducing a molecular weight of a starch to obtain a partially degraded starch;
   (a2) introducing an acidic group into the partially degraded starch obtained in (a1) to obtain a water-soluble polymer; and
   (a3) drying the water-soluble polymer at 70°C to 180°C in the presence of water so that it has a solid content of 90% or more.

2. The method for producing a water-absorbing resin according to claim 1, comprising, after (a2), (aa) desalting the water-soluble polymer.

3. The method for producing a water-absorbing resin according to claim 1 or 2, comprising, in (a2) or after (a2) but before (a3),
   (ab) partially neutralizing the acidic group in the water-soluble polymer.

4. The method for producing a water-absorbing resin according to claim 1 or 2, not comprising, after (a2) but before (a3), partially neutralizing the acidic group in the water-soluble polymer.

5. The method for producing a water-absorbing resin according to any one of claims 1 to 4, comprising, in (a3), drying the water-soluble polymer so that it has a solid content of at least 90% but not more than 99%.

6. The method for producing a water-absorbing resin according to any one of claims 1 to 5,
   wherein the acidic group is a carboxyalkyl group, a carboxyalkenyl group, or a sulfoalkyl group.

7. The method for producing a water-absorbing resin according to any one of claims 1 to 6, comprising, in (a1), obtaining the partially degraded starch that has at least one of a weight average molecular weight (Mw) of 7,500,000 or less or a dispersity (weight average molecular weight (Mw)/number average molecular weight (Mn)) of 5 or more.

8. The method for producing a water-absorbing resin according to any one of claims 1 to 7, comprising, in (a1), reducing the molecular weight of the starch by enzymatic treatment.

9. The method for producing a water-absorbing resin according to any one of claims 1 to 8, comprising, in (a2), introducing the acidic group into a mixture of the partially degraded starch obtained in (a1) and a starch whose molecular weight has not been reduced.

10. The method for producing a water-absorbing resin according to any one of claims 1 to 9,
    wherein the water-soluble polymer obtained in (a2) has a weight average molecular weight (Mw) of 500,000 to 40,000,000 as determined by aqueous size exclusion chromatography using pullulan standards.

11. The method for producing a water-absorbing resin according to any one of claims 1 to 10,
    wherein the water-absorbing resin to be produced has at least one of the following characteristics:

    (a) a water absorption rate under no pressure for ion-exchanged water is 100 to 500 g/g;
    (b) a water retention rate for ion-exchanged water is 80 to 300 g/g;
    (c) a water absorption rate under no pressure for physiological saline is 10 to 70 g/g; and
    (d) a water retention rate for physiological saline is 5 to 65 g/g.

FIG.1

Gel properties of water-absorbing resin

| Evaluation result | 1: Soluble in water | 2: Semi-soluble in water | 3: Insoluble in water and watery | 4: Insoluble in water and a little watery | 5: Insoluble in water and soft | 6: Insoluble in water and firm |
|---|---|---|---|---|---|---|
| Typical examples | (Completely in a liquid state and thus omitted) | 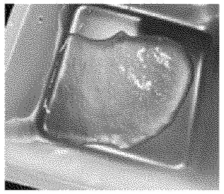 | 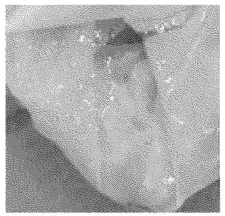 | 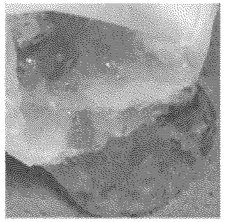 | 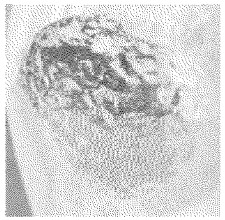 | 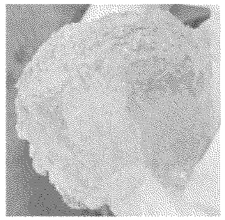 |

**INTERNATIONAL SEARCH REPORT**

International application No. **PCT/JP2023/013188**

**A. CLASSIFICATION OF SUBJECT MATTER**

***C08B 31/12***(2006.01)i; ***A61F 13/15***(2006.01)i; ***A61F 13/53***(2006.01)i; ***C08B 31/04***(2006.01)i
FI: C08B31/12; A61F13/53 300; A61F13/15 320; C08B31/04

According to International Patent Classification (IPC) or to both national classification and IPC

**B. FIELDS SEARCHED**

Minimum documentation searched (classification system followed by classification symbols)

C08B31/12; A61F13/15; A61F13/53; C08B31/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | US 6703496 B1 (KOERBER, Helmut) 09 March 2004 (2004-03-09) column 2, lines 50-62, columns 4-6, tables 1-4 | 1, 4, 5, 7-10 |
| A | US 5079354 A (KIMBERLY-CLARK CORPORATION) 07 January 1992 (1992-01-07) column 4, lines 52-68 | 1-11 |
| A | JP 2010-504414 A (ARCHER-DANIELS-MIDLAND COMPANY) 12 February 2010 (2010-02-12) | 1-11 |
| A | JP 8-89796 A (NIPPON SHOKUBAI CO LTD) 09 April 1996 (1996-04-09) | 1-11 |
| A | JP 8-208703 A (DEGUSSA AG) 13 August 1996 (1996-08-13) | 1-11 |
| A | JP 2007-222704 A (MITSUBISHI RAYON CO LTD) 06 September 2007 (2007-09-06) | 1-11 |
| A | JP 2012-12549 A (HARA, Toshio) 19 January 2012 (2012-01-19) | 1-11 |
| A | JP 9-504331 A (KIMBERLY-CLARK CORPORATION) 28 April 1997 (1997-04-28) | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 April 2023** | **16 May 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/013188**

**C. DOCUMENTS CONSIDERED TO BE RELEVANT**

| Category* | Citation of document, with indication, where appropriate, of the relevant passages | Relevant to claim No. |
|---|---|---|
| P, X | WO 2022/211000 A1 (NAGASE & CO., LTD.) 06 October 2022 (2022-10-06) claims, examples | 1-11 |
| P, X | WO 2022/211002 A1 (NAGASE & CO., LTD.) 06 October 2022 (2022-10-06) claims, examples | 1-11 |
| P, X | WO 2022/211005 A1 (NAGASE & CO., LTD.) 06 October 2022 (2022-10-06) claims, examples | 1-11 |

Form PCT/ISA/210 (second sheet) (January 2015)

**INTERNATIONAL SEARCH REPORT**
**Information on patent family members**

International application No.
**PCT/JP2023/013188**

| Patent document cited in search report | Publication date (day/month/year) | Patent family member(s) | Publication date (day/month/year) |
|---|---|---|---|
| US 6703496 B1 | 09 March 2004 | WO 2000/037505 A1<br>EP 1157044 A1<br>AU 2433000 A<br>CA 2357880 A | |
| US 5079354 A | 07 January 1992 | CA 2017676 A<br>MX 166639 B | |
| JP 2010-504414 A | 12 February 2010 | WO 2008/037082 A1<br>EP 2066699 A1<br>CA 2664392 A<br>CN 101541836 A<br>MX 2009003252 A<br>AU 2007302586 A<br>KR 10-2012-0117944 A | |
| JP 8-89796 A | 09 April 1996 | (Family: none) | |
| JP 8-208703 A | 13 August 1996 | EP 714913 A1<br>DE 4442605 A | |
| JP 2007-222704 A | 06 September 2007 | (Family: none) | |
| JP 2012-12549 A | 19 January 2012 | (Family: none) | |
| JP 9-504331 A | 28 April 1997 | US 5550189 A<br>WO 1995/011925 A1<br>EP 566118 A1<br>BR 9407917 A<br>SK 49996 A<br>AU 1084595 A<br>PL 317098 A<br>HU 75504 A<br>CA 2130427 A<br>CZ 122396 A<br>CZ 293534 B<br>CN 1139437 A<br>KR 10-0331902 B<br>MX 9301563 A | |
| WO 2022/211000 A1 | 06 October 2022 | (Family: none) | |
| WO 2022/211002 A1 | 06 October 2022 | (Family: none) | |
| WO 2022/211005 A1 | 06 October 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5079354 A
- JP 2010504414 T
- JP 2007222704 A

### Non-patent literature cited in the description

- *Synthesis*, 1989, vol. 12, 949-950